# EUROPÄISCHE PATENTSCHRIFT

(11) **EP 3 097 870 B1**
(45) Veröffentlichungstag und Bekanntmachung des Hinweises auf die Patenterteilung: **27.03.2019**
(21) Anmeldenummer: 16173579.0
(22) Anmeldetag: 17.12.2010
(51) Int. Cl.: A61B 17/11, A61B 18/14, A61B 17/115, A61B 18/00, A61B 17/072, A61B 18/12

(54) **CHIRURGISCHES SYSTEM ZUM VERBINDEN VON KÖRPERGEWEBE**
SURGICAL SYSTEM FOR CONNECTING BODILY TISSUES
SYSTEME CHIRURGICAL DESTINE A RELIER DES TISSUS CORPORELS

(30) Priorität: 17.12.2009 DE 102009059196
(43) Veröffentlichungstag der Anmeldung: 30.11.2016
(62) Teilanmeldung aus: 10195537.5
(73) Patentinhaber: Aesculap AG, 78532 Tuttlingen (DE)
(72) Erfinder: WEISSHAUPT, Dieter, 78194 Immendingen (DE); KELLER, Anton, 78589 Dürbheim (DE); ROTHWEILER, Christoph, 78166 Donaueschingen (DE)
(74) Vertreter: Winter, Brandl, Fürniss, Hübner, Röss, Kaiser, Polte - Partnerschaft mbB

(56) Entgegenhaltungen:
- US-A- 5 957 920
- US-A1- 2004 002 703
- US-A1- 2005 205 639
- US-A1- 2006 201 989
- US-A1- 2009 048 589

## Beschreibung

Die vorliegende Erfindung betrifft ein chirurgisches System zum Verbinden von Körpergewebe, umfassend ein chirurgisches Instrument mit zwei relativ zueinander bewegbaren Werkzeugelementen, welche jeweils eine HF-Elektrode umfassen, die in einer Annäherungsstellung der Werkzeugelemente einen minimalen Abstand voneinander definieren, einander gegenüberliegen und aufeinander zu weisen.

Chirurgische Systeme der eingangs beschriebenen Art können insbesondere genutzt werden, um Körpergewebeteile miteinander zu verbinden. Insbesondere dann, wenn bei End-zu-End-Anastomosen schlauchförmige Abschnitte eines Hohlorgans miteinander verbunden werden, kann es beim Herausführen des Instruments durch die hergestellte Gewebeverbindung hindurch zu einer Überdehnung derselben kommen, beispielsweise durch das zweite Werkzeugelement.

Aus US 5,957,920 A ist ein Instrument bekannt, das zur selektiven thermischen Zerstörung von Zellen um den zwischen der Blase und der Harnröhre liegenden Schließmuskel eines Patienten eingesetzt wird, um so die Zellstruktur und die biomechanischen Eigenschaften des Schließmuskels zu verändern. Hierfür sind kleine Elektrodenabschnitte an streifenförmigen Abschnitten angeordnet, die nach dem Einführen des Instruments zur Behandlung elastisch nach Außen gebogen werden können.

US 2004/0002703 A1 offenbart ein Instrument, das bei Menorrhagie zum Ablösen bzw. Zerstören der Gebärmutterschleimhaut mittels HF-Strom verwendet wird. Dabei werden Elektrodenbeine zunächst in einer zusammengefalteten Stellung eingeführt und in der Gebärmutter zu einem Dreieck aufgespannt, so dass diese mit dem Fundus und den Seitenwände der Gebärmutter in Kontakt kommen.

Beide Instrumente sind jedoch nicht zur Herstellung von End-zu-End-Anastomosen geeignet.

US2009/048589 offenbart ein chirurgisches System mit den Merkmalen des Oberbegriffs von Anspruch 1. Es ist daher Aufgabe der vorliegenden Erfindung, ein chirurgisches System der eingangs beschriebenen Art so zu verbessern, dass eine Überdehnung insbesondere mittels eines Stromflusses erzeugter Körpergewebeteilverbindungen beim Entfernen des chirurgischen Instruments vermieden wird.

Diese Aufgabe wird durch ein chirurgisches System gemäß Anspruch 1 gelöst, wobei das Instrument einen Schaft aufweist, an dessen distalem Ende mindestens ein erstes der Werkzeugelemente angeordnet oder ausgebildet ist, dass ein zweites Werkzeugelement von einer Betriebsstellung, in welcher es in die Annäherungsstellung bringbar ist, in eine Entfernungsstellung und/oder umkehrt bringbar ist, in welcher Entfernungsstellung ein Flächenbereich einer senkrechten Projektion des zweiten Werkzeugelementes auf eine Projektionsebene, welche senkrecht zur Schaftrichtung im Bereich des zweiten Werkzeugelements verläuft, kleiner ist als in der Betriebsstellung.

Das zweite Werkzeugelement von der Betriebsstellung in die Entfernungsstellung in der definierten Weise bringen zu können hat insbesondere den Vorteil, dass die erforderliche freie Fläche, um das zweite Werkzeugelement durch den Verbindungsbereich der Gewebeteile durchzuführen, in der Entfernungsstellung gegenüber der Betriebsstellung deutlich herabgesetzt ist. Insbesondere kann bei geeigneter Ausgestaltung des Systems sichergestellt werden, dass in der Entfernungsstellung keine Dehnung der frisch miteinander verbundenen Gewebeteile im Bereich von deren Verbindung erfolgen kann, wenn das Instrument aus den miteinander verbundenen Gewebeteilen entfernt wird. Dies wirkt sich auf End-zu-End-, Seit-zu-End- und Seit-zu-Seit-Anastomosen positiv aus. Insbesondere dann, wenn das zweite Werkzeugelement aus der Entfernungsstellung wieder in die Betriebsstellung gebracht werden kann, können mit dem Instrument auf einfache Weise auch mehrere Anastomosen nacheinander hergestellt werden, ohne dass das Instrument insgesamt aus den miteinander zu verbindenden schlauchförmigen Gewebeteilen entfernt werden muss.

Die Konstruktion des Systems lässt sich auf einfache Weise dadurch vereinfachen, dass das zweite Werkzeugelement ring- oder tellerförmig ausgebildet wird. Ferner können an derartigen zweiten Werkzeugelementen insbesondere auch ringförmige, das heißt in sich geschlossene, Elektroden einfach und sicher angeordnet werden.

Zum Überführen des zweiten Werkzeugelements von der Betriebsstellung in die Entfernungsstellung und/oder umkehrt ist es günstig, wenn das zweite Werkzeugelement an einem Halteglied bewegbar gelagert ist. Beispielsweise kann so das zweite Werkzeugelement in definierter Weise relativ zum Halteglied bewegt werden, das Halteglied optional selbst relativ zu einem weiteren Teil des Instruments, beispielsweise einem Schaft desselben.

Besonders einfache Konstruktionen für bewegbare Lagerungen des zweiten Werkzeugelements am Halteglied lassen sich beispielsweise realisieren, wenn das zweite Werkzeugelement am Halteglied verschieb- und/oder verschwenkbar gelagert ist.

Günstigerweise ist das zweite Werkzeugelement um eine Schwenkachse verschwenkbar gelagert, welche quer, insbesondere senkrecht, zu einer vom Halteglied definierten Haltegliedlängsachse verläuft. Beispielsweise kann so ein ring- oder tellerförmiges zweites Werkzeugelement, welches in der Betriebsstellung eine Ebene senkrecht zur Haltegliedlängsachse definiert, so verschwenkt werden, dass es in der Entfernungsstellung relativ zu der beschriebenen Ebene geneigt ist, insbesondere senkrecht zu dieser steht.

Um das zweite Werkzeugelement in der Entfernungsstellung in eine möglichst kompakte Form bringen zu können, ist es vorteilhaft, wenn das chirurgische System einen Faltmechanismus zum Überführen des zweiten Werkzeugelements von der Betriebsstellung in die Entfernungsstellung umfasst. Beispielsweise kann ein Faltmechanismus derart ausgebildet sein, dass das zweite Werkzeugelement selbst wiederum zwei Teile umfasst, die relativ zueinander verschwenk- oder anderweitig bewegbar angeordnet oder ausgebildet sind.

Um das zweite Werkzeugelement auf einfache Weise von der Betriebsstellung in die Entfernungsstellung und/oder umgekehrt bringen zu können, ist es günstig, wenn der Faltmechanismus ein Kraftübertragungselement zum Übertragen eine Betätigungskraft auf das zweite Werkzeugelement zum Überführen desselben von der Betriebsstellung in die Entfernungsstellung und/oder umgekehrt umfasst. Mit dem Kraftübertragungselement ist es so möglich, den Faltmechanismus zu betätigen, der ein Überführen des zweiten Werkzeugelements von der Betriebsstellung in die Entfernungsstellung und/oder umgekehrt ermöglicht.

Vorzugsweise ist das Kraftübertragungselement relativ zum Halteglied bewegbar angeordnet. Beispielsweise kann das zweite Werkzeugelement mittels des Halteglieds in eine gewünschte Position gebracht werden, wobei eine Betätigung des Faltmechanismus mittels des Kraftübertragungselements möglich ist, welches relativ zum Halteglied bewegt werden kann.

Je nach Einsatzzweck und Ausgestaltung des chirurgischen Systems kann es vorteilhaft sein, wenn das Kraftübertragungselement und das Halteglied relativ zueinander verschieb- und/oder verdreh- und/oder verschraubbar ausgebildet sind. Eine derartige Anordnung des Kraftübertragungselements und des Halteglieds relativ zueinander ermöglicht praktisch beliebige Betätigungsmechanismen vorzusehen, um zum Beispiel den Faltmechanismus zu betätigen.

Gemäß einer bevorzugten Ausführungsform kann vorgesehen sein, dass das Halteglied und das Kraftübertragungselement relativ zum Schaft bewegbar angeordnet sind. Beispielsweise kann so der Schaft von einem Operateur gehalten und das Kraftübertragungselement relativ zum Schaft bewegt werden, wodurch insbesondere der Faltmechanismus betätigt werden kann, um den zum Entfernen des zweiten Werkzeugelements benötigten Flächenbedarf zu verringern. Durch eine relative Bewegbarkeit des Halteglieds und des Schafts zueinander können die Werkzeugelemente relativ zueinander auch in der Betriebsstellung bewegt werden, wodurch sie beispielsweise zum Verbinden von Gewebe in eine Annäherungsstellung mit möglichst geringem Abstand bringbar sind und beispielsweise vor dem Überführen des Instruments von der Betriebsstellung in die Entfernungsstellung wieder voneinander weg bewegt werden können.

Gemäß einer weiteren bevorzugten Ausführungsform kann es vorteilhaft sein, wenn ein mit dem Faltmechanismus und/oder dem Kraftübertragungselement und/oder dem Halteglied gekoppelter Betätigungsmechanismus zum Betätigen des Faltmechanismus und/oder zum Bewegen des Kraftübertragungselements und/oder des Halteglieds relativ zum Schaft vorgesehen ist. Der so definierte Betätigungsmechanismus gestattet es, abhängig von der Ausbildung des Instruments, das zweite Werkzeugelement auf einfache und sichere Weise von der Betriebsstellung in die Entfernungsstellung und umgekehrt zu bringen.

Um eine Bewegung des zweiten Werkzeugelements und des Kraftübertragungselements zu erleichtern und/oder zu stabilisieren, ist es günstig, wenn das zweite Werkzeugelement und das Kraftübertragungselement über mindestens ein Anlenkglied miteinander gelenkig gekoppelt sind. Denkbar ist es auch, zwei, drei oder mehr Anlenkglieder vorzusehen.

Zum Verschwenken des zweiten Werkzeugelements relativ zum Halteglied ist es vorteilhaft, wenn das mindestens eine Anlenkglied mit einem Ende am zweiten Werkzeugelement an einem Angriffs- oder Gelenkpunkt angreift, welcher von der Schwenkachse beabstandet ist. Je nach Wahl des Angriffs- oder Gelenkpunkts kann so eine zum Verschwenken des zweiten Werkzeugelements erforderliche Kraft eingestellt werden.

Vorteilhafterweise ist das zweite Werkzeugelement zwei- oder mehrteilig ausgebildet. Je nach Gestaltung der das zweite Werkzeugelement ausbildenden Teile kann das zweite Werkzeugelement so in der Entfernungsstellung in eine besonders kompakte Form gebracht werden.

Gemäß einer weiteren bevorzugten Ausführungsform kann vorgesehen sein, dass das zweite Werkzeugelement mindestens zwei Werkzeugelementteile umfasst, welche relativ zueinander beim Übergang von der Betriebsstellung in die Entfernungsstellung bewegbar sind. Beispielsweise können sie relativ zueinander verschieb- und/oder rotierbar ausgebildet sein, um eine Fläche des zweiten Werkzeugelements in der Entfernungsstellung im Vergleich zur Betriebsstellung zu verringern.

Besonders einfach ausbilden lässt sich das chirurgische System, wenn die mindestens zwei Werkzeugelementteile relativ zueinander verschwenkbar sind. Insbesondere können sie so auf einfache Weise zusammengeklappt werden.

Um miteinander zu verbindende Gewebeteile mittels eines Stroms miteinander zu verbinden, was nachfolgend auch als Verschweißen oder Versiegeln bezeichnet wird, ist es günstig, wenn das zweite Werkzeugelement ein in Schaftrichtung bewegbares und in Richtung auf das erste Werkzeugelement hin und von diesem weg bewegbares Elektrodenelement umfasst. Insbesondere kann das Elektrodenelement eine HF-Elektrode tragen. Das Elektrodenelement selbst kann optional auch zwei- oder mehrteilig ausgebildet sein, um so einen Platzbedarf des zweiten Werkzeugelements in der Entfernungsstellung im Vergleich zur Betriebsstellung zu verringern.

Vorteilhaft ist es, wenn mindestens eine der HF-Elektroden in mindestens zwei Elektrodensegmente unterteilt ist und wenn die mindestens zwei Elektrodensegmente gegeneinander elektrisch isoliert sind. Die Unterteilung mindestens einer der HF-Elektroden in zwei oder mehr Elektrodensegmente hat insbesondere den Vorteil, dass sich die Verfahrensparameter zum Verbinden der miteinander zu verbindenden Gewebeteile signifikant leichter kontrollieren lassen. Je kleiner die Flächen, zwischen denen der HF-Strom zur Anwendung kommt, umso leichter lassen sich die Verfahrensparameter kontrollieren. Insbesondere haben die Temperatur, der Druck sowie die Gewebeimpedanz einen wesentlichen Einfluss auf das Verbindungsergebnis. Beispielsweise ist es so auch möglich, die Verfahrensparameter optimal auf die Gewebebeschaffenheit und insbesondere auch automatisch einzustellen. Darüber hinaus werden, anders als bei einem Klammernahtgerät, keine Klammern benötigt, die als Fremdkörper im Körper bleiben. Insbesondere ermöglichen die die HF-Elektrode beziehungsweise die HF-Elektroden unterteilenden Elektrodensegmente eine segmentweise Bestromung der HF-Elektrode, so dass die miteinander zu verbindenden Gewebeteile segmentweise miteinander verschweißt oder versiegelt werden können. Eine durch die Segmentierung der HF-Elektroden mögliche segmentweise Bestromung gestattet es, während des Verbindungs- oder Versiegelungsprozesses weniger Energie in die Gewebeteile einzubringen als bei vergleichbaren, unsegmentierten HF-Elektroden. Ferner hat die Segmentierung den Vorteil, dass zwischen durch HF-Bestromung verbundenen Bereichen der miteinander zu verbindenden Gewebeteile Gewebebereiche unverändert und im Wesentlichen ungeschädigt bleiben, so dass ausgehend von diesen neues Zellwachstum ermöglicht wird, welches zusätzlich zur durch den HF-Strom bewirkten Verbindung eine dauerhafte Verbindung der Gewebeteile durch ein Zusammenwachsen derselben ermöglicht.

Vorteilhaft ist es, wenn das erste Werkzeugelement eine in distaler oder im Wesentlichen in distaler Richtung weisende Randfläche des Schafts umfasst. Beispielsweise kann so auf einfache Weise ein distales Ende des Schafts gegen einen Gewebeteil gedrückt oder gehalten werden, der mit einem anderen Gewebeteil verbunden werden soll. Außerdem kann so auch einfach und sicher eine definierte Werkzeugelementfläche vorgegeben werden.

Günstig ist es, wenn am Schaft und/oder am ersten Werkzeugelement in Richtung auf das zweite Werkzeugelement hin weisende Kontaktglieder abstehen, welche in einer Gewebeverbindungsstellung mit den Elektrodensegmenten des zweiten Werkzeugelement in elektrisch leitenden Kontakt bringbar und in einer Gewebefassstellung von den Elektrodensegmenten des zweiten Werkzeugelements beabstandet sind. Mit den Kontaktgliedern ist es möglich, die Elektrodensegmente des zweiten Werkzeugelements zu kontaktieren und durch eine beispielsweise im Schaft vorgesehene elektrisch leitende Verbindung an eine Stromquelle, beispielsweise einen HF-Generator, anzuschließen. Ferner hat die vorgeschlagene Ausbildung den Vorteil, dass lediglich in der Gewebeverbindungsstellung ein Kontakt zwischen den Elektrodensegmenten des zweiten Werkzeugelements und den Kontaktgliedern herstellbar ist, so dass in der Gewebefassstellung die Elektrodensegmente des zweiten Werkzeugelements nicht versehentlich bestromt werden können. Die Handhabung des chirurgischen Systems wird dadurch insgesamt noch sicherer.

Damit die Werkzeugelemente auf einfache Weise relativ zueinander bewegt werden können, ist es günstig, wenn das Instrument eine Betätigungseinrichtung umfasst zum Bewegen der Werkzeugelemente relativ zueinander. Günstigerweise ist mindestens eine der HF-Elektroden in eine Mehrzahl von Elektrodensegmenten unterteilt. Unter einer Mehrzahl von Elektrodensegmenten sind im Sinne dieser Anmeldung mehr als zwei Elektrodensegmente zu verstehen, die eine noch weiter verbesserte Kontrollierbarkeit der Verfahrensparameter ermöglichen.

Vorteilhafterweise bilden in der Annäherungsstellung einander gegenüberliegende und aufeinander zu weisende Elektrodensegmente ein Elektrodensegmentpaar. Ein derartiges Elektrodensegmentpaar kann beispielsweise als Einheit angesteuert werden. Auf diese Weise können insbesondere lokale Randbedingungen im Bereich der beiden Elektrodensegmente optimal berücksichtigt werden, insbesondere Temperatur, Druck und die Gewebeimpedanz von zwischen dem Elektrodensegmentpaar gehaltenem Gewebe.

Um den HF-Strom in besonders definierter Weise zum Verbinden des Gewebes von einem Elektrodensegment des Elektrodensegmentpaars zum zugehörigen Elektrodensegment leiten zu können, ist es günstig, wenn die das Elektrodensegmentpaar bildenden Elektrodensegmente geometrisch ähnlich sind.

Weiter verbessern lässt sich die Funktion des Systems, beispielsweise dadurch, dass die das Elektrodensegmentpaar bildenden Elektrodensegmente gleich groß oder im Wesentlichen gleich groß sind. Auf diese Weise können insbesondere Stromdichten optimal vorgegeben werden.

Besonders einfach ausbilden lassen sich die mindestens zwei Elektrodensegmente, wenn sie streifenförmig oder im Wesentlichen streifenförmig ausgebildet sind.

Gemäß einer bevorzugten Ausführungsform kann vorgesehen sein, dass jedes der Werkzeugelemente eine Werkzeugelementfläche definiert und dass die HF-Elektrode einen Teil der Werkzeugelementfläche bildet. Diese Ausbildung ermöglicht es, die Werkzeugelemente praktisch vorsprungsfrei auszubilden.

Günstig ist es, wenn die Werkzeugelementfläche in der Betriebsstellung eine Projektionsfläche definiert oder parallel zu dieser verläuft. Zum Beispiel kann die Projektionsfläche durch die senkrechte Projektion definiert sein. Insbesondere kann so miteinander zu verbindendes Gewebe gleichmäßig gehalten und gegeneinander gedrückt werden, wenn insbesondere das erste Werkzeugelement eine in distaler Richtung weisende Stirnkante eines Schafts des Instruments bildet.

Vorzugweise ist die Werkzeugelementfläche eben. Dadurch wird die Herstellung des Instruments sowie dessen Reinigbarkeit deutlich vereinfacht.

Je nach Einsatzzweck des chirurgischen Systems, das heißt insbesondere in Abhängigkeit der zu verbindenden Gewebeteile, kann es günstig sein, wenn die Werkzeugelementfläche rechteckig oder ringförmig ausgebildet ist. Insbesondere eine ringförmige Werkzeugelementfläche ermöglicht es auf einfache Weise, End-zu-End-Anastomosen durchzuführen.

Vorteilhaft ist es, wenn die mindestens zwei Elektrodensegmente in mindestens zwei Elektrodenreihen nebeneinander angeordnet sind. Mindestens zwei Elektrodenreihen ermöglichen es mindestens zwei nebeneinander verlaufende Verbindungslinien herzustellen. Dadurch kann eine verbesserte Verbindung und insbesondere eine optimale Abdichtung der Verbindungsstelle zwischen den Gewebeteilen erreicht werden. Insbesondere können zwischen den Elektrodenreihen auch noch nach dem Verbinden der Gewebeteile durch HF-Bestromung vollständig oder im Wesentlichen ungeschädigte Zellen erhalten bleiben, von denen neues Zellwachstum ausgehen kann. Dies ermöglicht zusätzlich zur Verbindung der Gewebeteile durch Verschweißen langfristig eine dauerhafte Verbindung der Gewebeteile durch Zusammenwachsen intakter Zellen.

Vorzugweise umfasst jede Elektrodenreihe mindestens zwei Elektrodensegmente, die elektrisch gegeneinander isoliert sind. So kann zumindest eine sequentielle Bestromung realisiert werden.

Gemäß einer weiteren bevorzugten Ausführungsform kann vorgesehen sein, dass mindestens ein Elektrodensegment einen ersten Elektrodensegmentabschnitt, welcher Teil einer ersten Elektrodenreihe ist, und einen zweiten Elektrodensegmentabschnitt aufweist, welcher Teil einer zweiten Elektrodenreihe ist. Auf diese Weise kann eine zweireihige Gewebeverbindung erzeugt werden, insbesondere zwei Verbindungslinien umfassend oder definierend, wobei durch die besonders ausgestalteten Elektrodensegmentabschnitte eine noch bessere Überlappung zwischen beiden Verbindungslinien erreicht wird, was insbesondere in einer verbesserten Abdichtung der Gewebeverbindung resultiert.

Um Gewebe ringförmig miteinander verbinden zu können, was insbesondere für End-zu-End-Anastomosen erforderlich ist, ist es günstig, wenn die mindestens zwei Elektrodenreihen in sich geschlossen ringförmig ausgebildet sind.

Damit jedes Elektrodensegment bei Bedarf individuell bestromt werden kann, ist es vorteilhaft, wenn jedes Elektrodensegment mit einem Anschlusskontakt elektrisch leitend verbunden ist. Der Anschlusskontakt kann wiederum mit anderen Anschlusskontakten verbunden oder direkt mit einer Stromquelle verbunden oder verbindbar sein.

Gemäß einer weiteren bevorzugten Ausführungsform kann vorgesehen sein, dass die mindestens eine in mindestens zwei Elektrodensegmente unterteilte HF-Elektrode eine Elektrodenlänge definiert und dass jedes der mindestens zwei Elektrodensegmente eine Segmentlänge definiert, welche kleiner ist als die Elektrodenlänge. Durch diese Konstruktion kann insbesondere sichergestellt werden, dass mit jedem Elektrodensegment nur ein Abschnitt der miteinander zu verbindenden Gewebeteile verbunden werden kann, welcher kleiner ist als eine Gesamtlänge der HF-Elektrode.

Zur Verbesserung einer Dichtheit einer mittels des chirurgischen Systems hergestellten Verbindungsstelle zwischen zwei Gewebeteilen ist es günstig, wenn die Summe aller Segmentlängen größer ist als die Elektrodenlänge. Dies gewährleistet zumindest teilweise eine Überlappung von mit den Elektrodensegmenten hergestellten Gewebeverbindungen.

Um das Instrument einfach und sicher mit einem HF-Generator oder mit einer anderen geeigneten HF-Stromquelle verbinden zu können, ist es günstig, wenn das Instrument mindestens zwei HF-Anschlusskontakte umfasst, welche elektrisch leitend mit den mindestens zwei Elektrodensegmenten verbunden oder verbindbar sind.

Um Gewebe zwischen den beiden Werkzeugelementen fassen und gegebenenfalls während des Verbindungsprozesses halten zu können, ist es vorteilhaft, wenn die Werkzeugelemente relativ zueinander verschwenkbar und/oder verschiebbar ausgebildet sind. Insgesamt ist also eine bewegbare Anordnung der Werkzeugelemente relativ zueinander wünschenswert.

Um die Handhabbarkeit des chirurgischen Instruments weiter zu verbessern, ist die Betätigungseinrichtung vorzugsweise an einem proximalen Ende des Instruments angeordnet oder ausgebildet. Beispielsweise dann, wenn das Instrument einen Schaft aufweist, kann dieser durch eine Körperöffnung ins Innere des Körpers eingeführt werden, wobei dann die Werkzeugelemente relativ zueinander mittels der Betätigungseinrichtung betätigbar sind, die dabei vorzugsweise noch aus dem Körper des Patienten herausragt. Insgesamt kann so auf einfache Weise ein endoskopisches oder minimalinvasives chirurgisches Instrument ausgebildet werden.

Die Handhabbarkeit des Instruments kann insbesondere dadurch für einen Operateur verbessert werden, dass die Betätigungseinrichtung zwei relativ zueinander verschwenkbare Betätigungsglieder umfasst, welche mit mindestens einem der Werkzeugelemente in Wirkverbindung stehen zum Übertragen einer Betätigungskraft zum Bewegen des mindestens einen Werkzeugelements relativ zum anderen Werkzeugelement. Die Betätigungsglieder können auch grundsätzlich nur relativ zueinander bewegbar ausgebildet sein, das heißt alternativ beispielsweise zu einer verschwenkbaren Anordnung können sie auch verschiebbar, oder verschwenk- und verschiebbar, zueinander angeordnet sein.

Gemäß einer weiteren bevorzugten Ausführungsform ist vorteilhafterweise vorgesehen, dass das Instrument ein HF-Schneidelement zum Durchtrennen von Gewebe umfasst. Das Vorsehen eines HF-Schneidelements, das beispielsweise Teil einer Schneideinrichtung des Instruments sein kann, gestattet es insbesondere, miteinander verbundene Gewebeteile in gewünschter Weise zu präparieren. Zum Beispiel kann dies der Fall sein, wenn End-zu-End-Anastomosen mit dem System erzeugt werden, wobei freie Enden schlauchförmigen Gewebes mittels des Instruments ringförmig verbunden und dann überstehendes Gewebe mittels des Schneidelements oder der Schneideinrichtung abgetrennt werden kann.

Vorzugweise weist das HF-Schneidelement eine Schneidkante auf, welche eine relativ zu einer Längsachse des Instruments, insbesondere im Bereich des HF-Schneidelements, geneigte Schneidebene definiert. Durch die geneigte Schneidebene kann beispielsweise ein HF-Strom über das Schneidelement geleitet werden, um Gewebe zu durchtrennen. Die derart ausgebildete Schneidkante weist dann lediglich in einem kleinen Bereich einen minimalen Abstand zu einer Gegenelektrode auf, die eine Ebene quer zur Längsachse des Instruments definiert. So kann ein Schneidfunken definiert erzeugt werden im Bereich des geringsten Abstands zwischen dem HF-Schneidelement und einer entsprechenden Gegenelektrode, wobei der Schneidfunke dann entlang der geneigten Schneidkante in definierter Weise wandern kann.

Um einen ringförmigen Schnitt einfach und sicher durchführen zu können, ist die Schneidkante günstigerweise ringförmig geschlossen.

Damit das HF-Schneidelement in definierter Weise mit einem HF-Strom beaufschlagt werden kann, ist es vorteilhaft, wenn das Instrument einen mit dem HF-Schneidelement elektrisch leitend verbundenen HF-Schneidanschluss aufweist. Insbesondere kann das HF-Schneidelement bei einer solchen Ausgestaltung definiert mit einen HF-Strom zum Durchtrennen von Gewebe beaufschlagt werden, vorzugsweise unabhängig und zeitlich getrennt von einer Beaufschlagung der Elektrodensegmente zum Verbinden der Gewebeteile miteinander mit einem HF-Strom.

Vorteilhaft ist es, wenn das Schneidelement relativ zu mindestens einem der Werkzeugelemente bewegbar angeordnet ist. Dies gestattet ist, beispielsweise das Schneidelement relativ zu den Werkzeugelementen so zu bewegen, dass es nicht mit den miteinander zu verbindenden Gewebeteilen in Kontakt kommen kann, wenn diese mittels der an den Werkzeugelementen ausgebildeten Elektrodensegmente miteinander verbunden werden. Vielmehr ist es so zum Beispiel möglich, das Schneidelement erst nach dem Verbinden der Gewebeteile in eine Position zu bringen, in der diese in gewünschter Weise beschnitten und/oder ganz oder teilweise durchtrennt werden können.

Um das HF-Instrument in gewünschter Weise mit einem HF-Strom beaufschlagen zu können, umfasst das chirurgische System vorzugsweise mindestens einen HF-Stromgenerator, welcher mit den HF-Elektroden und/oder dem Schneidelement wahlweise elektrisch leitend verbindbar ist. Insbesondere kann so der jeweils für das Verbinden beziehungsweise Durchtrennen von Gewebe optimale Strom eingestellt werden.

Gemäß einer weiteren bevorzugten Ausführungsform kann vorgesehen sein, dass das System mindestens eine Steuer- und/oder Regelungseinrichtung mit einer Schalteinrichtung zum sequentiellen Beaufschlagen der Elektrodensegmente mindestens einer HF-Elektrode mit HF-Strom umfasst. Optional kann mit der Steuer- und/oder Regelungseinrichtung auch eine weitere HF-Elektrode mit HF-Strom beaufschlagt werden. Durch die in der beschriebenen Weise ausgebildete Schalteinrichtung können insbesondere die Elektrodensegmente einer HF-Elektrode nacheinander, also in einer sequentiellen Abfolge, mit einem HF-Strom beaufschlagt werden, um die miteinander zu verbindenden Gewebeteile abschnittweise zu verbinden.

Günstig ist es, wenn das chirurgische System eine Steuer- und/oder Regelungseinrichtung mit einer Schalteinrichtung zum gleichzeitigen Beaufschlagen von mindestens zwei Elektrodensegmenten mindestens einer HF-Elektrode mit HF-Strom umfasst. Auf diese Weise kann der Verbindungs- oder Versiegelungsprozess beschleunigt oder schneller durchgeführt werden, da gleichzeitig zwei miteinander zu verbindende Gewebeteile längs zweier Abschnitte miteinander verbunden werden können. Insbesondere ist es auch denkbar, jeweils zwei Elektrodensegmente gleichzeitig und weitere Elektrodensegmente dann sequentiell mit einem HF-Strom zu beaufschlagen.

Um Kurzschlüsse zu vermeiden, wenn zwei Elektrodensegmente gleichzeitig mit HF-Strom beaufschlagt werden, ist es günstig, wenn zwischen den mindestens zwei Elektrodensegmenten mindestens ein weiteres Elektrodensegment angeordnet ist.

Günstig ist es, wenn die Schalteinrichtung ausgebildet ist zum Schalten mindestens eines HF-Ausgangs des mindestens einen Stromgenerators. Es können auch zwei, drei oder noch mehr HF-Ausgänge vorgesehen sein, die von der Schalteinrichtung gesteuert und/oder geregelt werden können, um beispielsweise gezielt einzelne Elektrodensegmente der HF-Elektroden mit einem HF-Strom gewünschter Stärke zu beaufschlagen.

Vorteilhaft ist es, wenn das chirurgische System einen HF-Generator umfasst, welcher mit den HF-Elektroden oder dem Schneidelement wahlweise elektrisch leitend verbindbar ist und die Steuer- und/oder Regelungseinrichtung umfasst. Auf diese Weise können mehrere Funktionen des Systems in einem Gerät untergebracht werden, was sowohl dessen Herstellung als auch dessen Handhabbarkeit verbessert.

Günstigerweise ist die Steuer- und/oder Regelungseinrichtung derart ausgebildet, dass eine Bestromungsstärke und/oder eine Bestromungsdauer für die einzelnen Elektrodensegmente einstellbar ist. Auf diese Weise können mittels der Steuer- und/oder Regelungseinrichtung insbesondere Verfahrensparameter wie Temperatur, Druck sowie Gewebeimpedanz direkt beziehungsweise indirekt im gewünschten Bereich gehalten werden.

Um eine zu starke Erwärmung der miteinander zu verbindenden Gewebeteile zu vermeiden, welche eine Zerstörung von Zellen zur Folge hätte, ist es vorteilhaft, wenn die Steuer- und/oder Regelungseinrichtung eine Temperaturmesseinrichtung umfasst zum Messen einer Elektrodensegmenttemperatur und/oder einer Gewebetemperatur.

Günstig ist es ferner, wenn die Steuer- und/oder Regelungseinrichtung eine Impedanzmesseinrichtung umfasst zum Messen einer Gewebeimpedanz von zwischen den Werkzeugelementen gehaltenem Gewebe. Die Bestimmung der Gewebeimpedanz bietet die Möglichkeit, abhängig von ihrem Wert den Strom- oder HF-Generator zu regeln, insbesondere die von diesem bereitgestellte Leistung. Auf diese Weise kann die zum Verbinden der Gewebeteile in diese einzubringende Energie einfach und sicher geregelt werden. Zur Messung der Gewebeimpedanz können insbesondere die HF-Elektroden verwendet werden. Eine Messung kann auch zwischen einzelnen Elektrodensegmenten erfolgen, die einander gegenüberliegen. Vorzugsweise wird die Gewebeimpedanz gemessen, wenn die HF-Elektroden gerade unbestromt sind. Insbesondere ist es günstig, die Gewebeimpedanz in den Pausen beim Umschalten der Polarität des HF-Stroms zu messen. Damit kann die Veränderung des Gewebes gut und praktisch in Echtzeit überwacht und weiterer Energieeintrag unterbunden oder gezielt weiter gestattet werden.

Die nachfolgende Beschreibung bevorzugter Ausführungsformen dient im Zusammenhang mit der Zeichnung der näheren Erläuterung. Es zeigen:
Figur 1: eine schematische Gesamtansicht eines chirurgischen Instruments zum Verbinden von Körpergewebeteilen;
Figur 2: eine vergrößerte, perspektivische, teilweise geschnittene und durchbrochene Ansicht des Bereichs A in Figur 1;
Figur 3: eine Längsschnittansicht des Instruments aus Figur 1 im Bereich A vor dem Verbinden zweier schlauchförmiger Gewebeteile;
Figur 4: eine Ansicht analog Figur 3 beim Verschweißen der Gewebeteile zur Herstellung einer End-zu-End-Anastomose;
Figur 5: eine Draufsicht auf eine Werkzeugelementfläche mit einer in vier Elektrodensegmente unterteilten HF-Elektrode;
Figur 6: eine perspektivische, schematische Ansicht eines zweiten Ausführungsbeispiels eines chirurgischen Instruments zum Verbinden von Körpergewebeteilen;
Figur 7: eine Draufsicht auf eine schematisch dargestellte Werkzeugelementfläche des Instruments aus Figur 6 in Richtung des Pfeils B;
Figur 8: eine schematische Ansicht ähnlich Figur 2 einer alternativen Ausgestaltung des Instruments in einer Gewebefassstellung;
Figur 9: eine Ansicht entsprechend Figur 8 des dort dargestellten Instruments mit teilweise abgeklapptem zweiten Werkzeugelement;
Figur 10: eine Schnittansicht längs Linie 10-10 in Figur 8;
Figur 11: eine schematische Schnittansicht ähnlich Figur 10 des in einer wie in Figur 9 dargestellten Stellung zusammengeklappten, zweiten Werkzeugelements;
Figur 12: eine alternative Ausführungsform eines zweiten Werkzeugelements in perspektivischer schematischer Darstellung;
Figur 13: eine Explosionsdarstellung eines Teils des in Figur 12 dargestellten zweiten Werkzeugelements;
Figur 14: eine Schnittansicht längs Linie 14-14 in Figur 12;
Figur 15: eine schematische Schnittansicht analog Figur 14 des dort dargestellten Ausführungsbeispiels mit teilweise abgeklapptem zweiten Werkzeugelement;
Figur 16: eine perspektivische schematische Darstellung ähnlich Figur 12 eines weiteren Ausführungsbeispiels eines zweiten Werkzeugelements;
Figur 17: eine vergrößerte Darstellung des zweiten Werkzeugelements aus Figur 16 in einer teilweise geneigten Stellung;
Figur 18: eine Schnittansicht längs Linie 18-18 in Figur 16; und
Figur 19: eine Ansicht analog Figur 18 bei teilweise geneigtem zweiten Werkzeugelement in einer Stellung, wie sie in Figur 17 dargestellt ist.

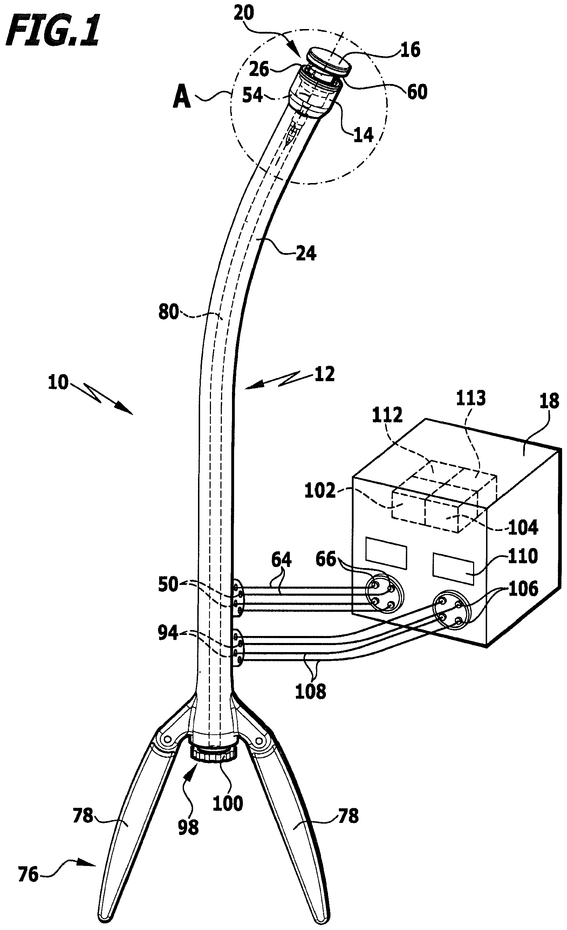

In Figur 1 ist ein chirurgisches System zum Verbinden von Körpergewebe schematisch dargestellt und insgesamt mit dem Bezugszeichen 10 bezeichnet. Es umfasst ein chirurgisches Instrument 12 mit zwei relativ zueinander bewegbaren Werkzeugelementen 14 und 16. Ferner umfasst das System 10 einen Stromgenerator in Form eines HF-Stromgenerators 18, welcher in einer weiter unten näher beschriebenen Weise mit dem Instrument 12 verbunden werden kann.

Die Werkzeugelemente 14 und 16 bilden einen Teil einer insgesamt mit dem Bezugszeichen 20 versehenen Verbindungseinrichtung zum Verbinden von Körpergewebe. Das erste Werkzeugelement 14 umfasst eine in distaler Richtung weisende Randfläche 22 eines langgestreckten, hülsenförmigen Schafts 24 des Instruments 12. Somit ist das erste Werkzeugelement an einem distalen Ende 26 des Instruments 12 angeordnet beziehungsweise ausgebildet.

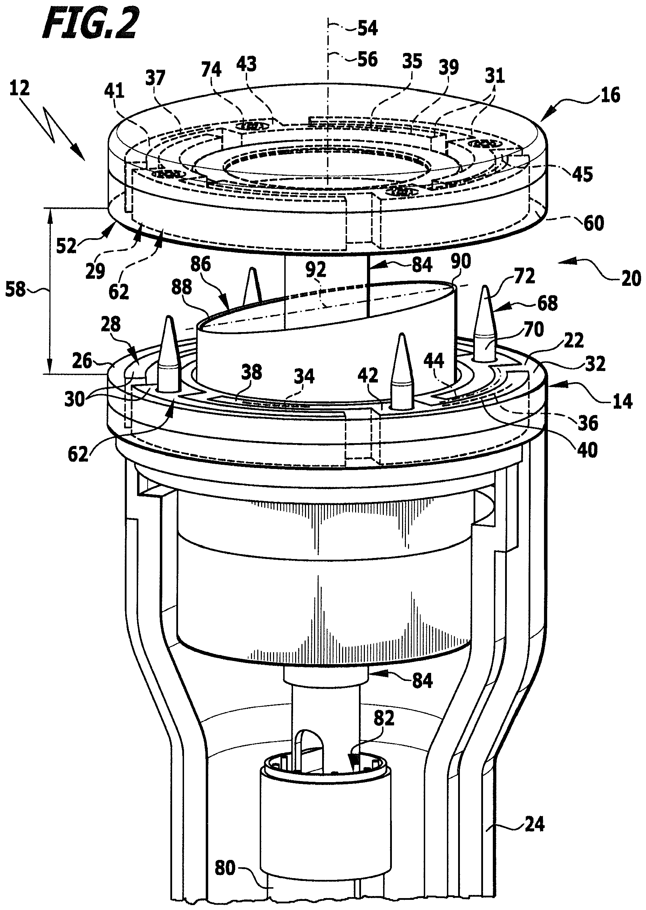

Das erste Werkzeugelement 14 umfasst eine HF-Elektrode 28. Sie ist in mindestens zwei, bei dem in den Figuren 2 bis 5 schematisch dargestellten Ausführungsbeispiel in vier Elektrodensegmente 30 unterteilt ist, die elektrisch gegeneinander isoliert sind. Die Elektrodensegmente 30 sind streifenförmig oder im Wesentlichen streifenförmig ausgebildet. Das erste Werkzeugelement 14 definiert eine Werkzeugelementfläche 32 derart, dass die HF-Elektrode 28 einen Teil derselben bildet. Insgesamt ist die Werkzeugelementfläche 32 eben und ringförmig ausgebildet.

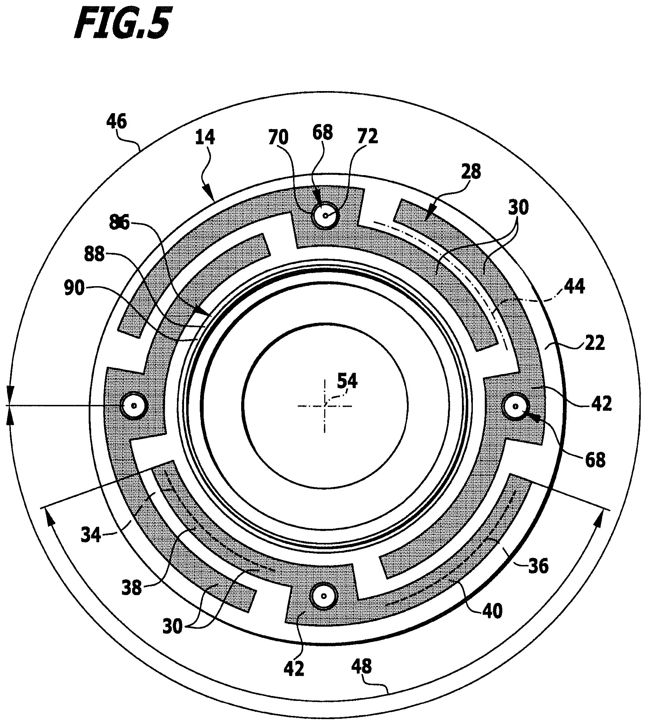

Die vier Elektrodensegmente 30 definieren zwei Elektrodenreihen 34 und 36. Jede Elektrodenreihe umfasst dabei jeweils einen Teil der vier Elektrodensegmente 30. Wie zum Beispiel in Figur 5 zu erkennen, weist jedes Elektrodensegment 30 einen ersten Elektrodensegmentabschnitt 38 auf, welcher einen Teil der ersten Elektrodenreihe 34 bildet, und einen zweiten Elektrodensegmentabschnitt 40, welcher einen Teil der zweiten Elektrodenreihe 36 bildet. Die beiden Elektrodenreihen 34 und 36 sind insgesamt gekrümmt ausgebildet, wobei die Elektrodensegmentabschnitte 38 und 40 jeweils elektrisch leitfähige Kreisringabschnitte definieren. Insgesamt sind die mindestens zwei Elektrodenreihen, die durch jeweils vier Elektrodensegmentabschnitte 38 beziehungsweise 40 definiert werden, in sich geschlossen ringförmig ausgebildet. Um die Elektrodensegmente 30 in gewünschter Weise kontaktieren zu können, ist jedes Elektrodensegment 30 mit einem Anschlusskontakt 42 elektrisch leitend verbunden, welcher in einem Verbindungsbereich zwischen den Elektrodensegmentabschnitten 38, 40 angeordnet ist. Zwischen den Elektrodenreihen bleiben auch noch nach dem Verbinden der Gewebeteile durch HF-Bestromung vollständig oder im Wesentlichen ungeschädigte Zellen erhalten, von denen neues Zellwachstum ausgehen kann. Dies ermöglicht zusätzlich zur Verbindung der Gewebeteile durch Verschweißen langfristig eine dauerhafte Verbindung der Gewebeteile durch Zusammenwachsen intakter Zellen.

Die HF-Elektrode 28 definiert eine zwischen den Elektrodensegmentabschnitten 38 und 40 verlaufende Elektrodenmittellinie 44. Aneinander angrenzende Elektrodensegmente 30 sind daher in einer von der Elektrodenmittellinie 44 definierten Richtung versetzt zueinander angeordnet. Insgesamt definiert die in vier Elektrodensegmente 30 unterteilte HF-Elektrode 28 eine Elektrodenlänge 46, wobei jede der vier Elektrodensegmente 30 eine Segmentlänge 48 definiert, welche kleiner ist als die Elektrodenlänge 46. Wie beispielhaft in Figur 5 dargestellt erstrecken sich die Elektrodensegmente 30 über einen Winkelbereich von etwa 140° und weisen damit eine Länge auf, die annähernd 40% der Elektrodenlänge 46 entspricht. Damit ist aber auch die Summe aller Segmentlängen 48 etwa um den Faktor 1,6 größer als die Elektrodenlänge 46.

Im Bereich eines proximalen Endes des Schafts 24 sind HF-Anschlusskontakte 50 angeordnet, die elektrisch leitend, zum Beispiel über im Schaft verlaufende Leitungen, mit den Elektrodensegmenten 30 verbunden sind. Vorzugsweise entspricht die Zahl der HF-Anschlusskontakte 50 der Zahl der Elektrodensegmente 30, also vier HF-Anschlusskontakte 50 für die vier Elektrodensegmente 30 des ersten Werkzeugelements 14.

Das zweite Werkzeugelement 16 ist im Wesentlichen scheibenförmig ausgebildet und umfasst ein Elektrodenelement 52, welches in Richtung auf das erste Werkzeugelement 14 hin und von diesem weg bewegbar ist, und zwar parallel zu einer Längsachse 54 des Schafts 24 im Bereich der Werkzeugelemente 14, 16, welche eine Schaftrichtung 56 definiert. Die Werkzeugelemente 14, 16 sind relativ zueinander verschiebbar angeordnet, das heißt ein Abstand 58 zwischen der Werkzeugelementfläche 32 des ersten Werkzeugelements 14 und einer Werkzeugelementfläche 60 des zweiten Werkzeugelements 16 ist veränderbar.

Das Elektrodenelement 52 umfasst eine HF-Elektrode 29, die in ihrem Aufbau der HF-Elektrode 28 entspricht. Dies bedeutet, dass sie ebenfalls vier Elektrodensegmente 31 umfasst, die nicht über die Werkzeugelementfläche 60 vorstehen. Es werden ebenfalls zwei Elektrodenreihen 35 und 37 definiert, wobei erste Elektrodensegmentabschnitte 39 die Elektrodenreihe 35 definieren und zweite Elektrodensegmentabschnitte 41 die Elektrodenreihe 37. Anschlusskontakte 43 sind ebenfalls vorgesehen, die jeweils einen Elektrodensegmentabschnitt 39 mit einem Elektrodensegmentabschnitt 41 leitend verbinden zur Ausbildung eines Elektrodensegments 31. Die HF-Elektroden 28 und 29 sind spiegelsymmetrisch zu einer senkrecht zur Längsachse 54 zwischen den Werkzeugelementflächen 32 und 60 verlaufenden Spiegelebene ausgebildet. Auf diese Weise werden Elektrodensegmentpaare 62 definiert von jeweils einem Elektrodensegment 30 und dem entsprechenden, gegenüberliegenden Elektrodensegment 31. Insgesamt umfasst das in den Figuren 1 bis 5 dargestellte Ausführungsbeispiel somit vier Elektrodensegmentpaare 62. Die Elektrodensegmente 30, 31 sind nicht nur geometrisch ähnlich, sondern gleich groß beziehungsweise im Wesentlichen gleich groß.

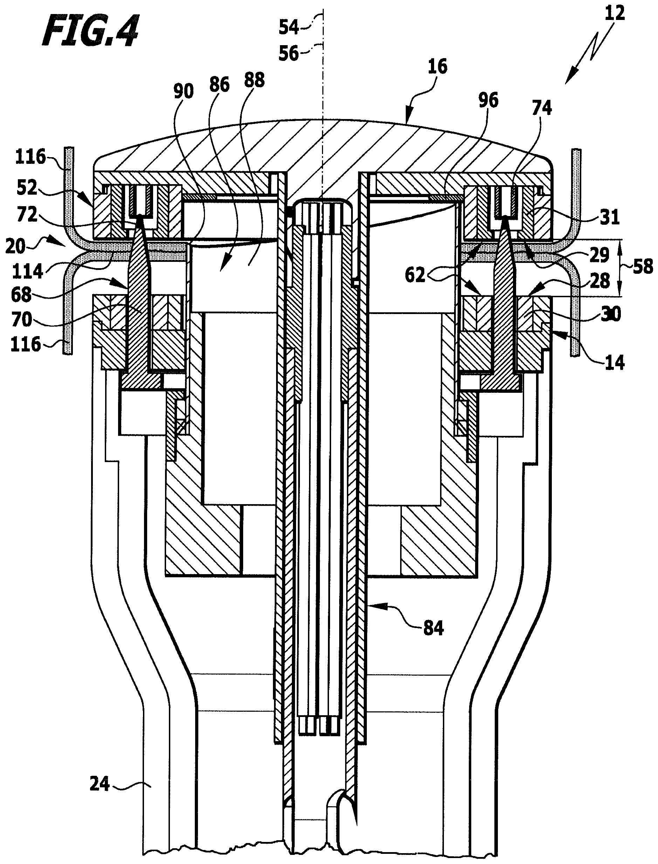

In einer Annäherungsstellung der Werkzeugelemente 14, 16 definieren die HF-Elektroden 28, 29 einen minimalen Abstand 58 voneinander. Die Annäherungsstellung ist schematisch in Figur 4 dargestellt. In der Annäherungsstellung liegen die HF-Elektroden 28 und 29 einander gegenüber und weisen aufeinander zu.

Die Elektrodensegmente 31 sind elektrisch leitend mit weiteren vier HF-Anschlusskontakten 50 verbindbar, von denen der Übersichtlichkeit wegen in Figur 1 lediglich zwei dargestellt sind. Mittels entsprechender Verbindungsleitungen 64 können die HF-Anschlusskontakte 50 mit entsprechenden Kontakten 66 des HF-Stromgenerators 18 verbunden werden. Wie bereits dargelegt, sind die HF-Anschlusskontakte 50 mit den Elektrodensegmenten 30 direkt elektrisch leitend verbunden. Um die HF-Anschlusskontakte 50 mit den Elektrodensegmenten 31 verbinden zu können, sind am Schaft 24 beziehungsweise am ersten Werkzeugelement 14 in Richtung auf das zweite Werkzeugelement 16 hin weisende Kontaktglieder 68 abstehend angeordnet, welche einen kurzen zylindrischen Abschnitt 70 und einen, ein freies Ende definierenden kegelförmigen Abschnitt 72 aufweisen. In einer Gewebeverbindungsstellung, wie sie beispielsweise schematisch in Figur 4 dargestellt ist, also in einer Stellung, in der sich die Werkzeugelemente 14 und 16 in der Annäherungsstellung befinden, ragen die freien Enden der Abschnitte 72 der Kontaktglieder 68 in entsprechende buchsenförmige Aufnahmen 74 des Elektrodenelements 52 hinein und stehen mit diesen elektrisch leitend in Kontakt. Die Kontaktglieder 68 wiederum sind längs des Schafts 24 über nicht dargestellte elektrische Leitungen mit den HF-Anschlusskontakten 50 verbunden. Die Aufnahmen 74 wiederum stehen mit den Anschlusskontakten 43 elektrisch leitend in Verbindung. Auf diese Weise kann in der Annäherungs- oder Gewebeverbindungsstellung auch ein elektrisch leitender Kontakt zwischen den HF-Anschlusskontakten 50 und den Elektrodensegmenten 31 hergestellt werden.

Selbstverständlich sind die Kontaktglieder 68, die die Elektrodensegmente 30 im Bereich von deren Anschlusskontakten 42 durchsetzen, gegenüber diesen isoliert, so dass keine Kurzschlüsse auftreten können. Zu diesem Zweck sind die Abschnitte 70 der Kontaktglieder 68 vorzugsweise mit einer elektrisch isolierenden Beschichtung oder Hülle versehen.

Um die Werkzeugelemente 14, 16 des Instruments 12 relativ zueinander bewegen zu können, ist eine Betätigungseinrichtung 76 an einem proximalen Ende oder Endbereich des Instruments 12 angeordnet. Die Betätigungseinrichtung 76 umfasst zwei relativ zueinander verschwenkbare Betätigungsglieder 78, welche mit einem im Inneren des Schafts bewegbar gelagerten Kraftübertragungsglied 80 beweglich gekoppelt sind, so dass dieses in Folge einer Verschwenkbewegung der Betätigungsglieder 78 in distaler beziehungsweise proximaler Richtung bewegbar ist.

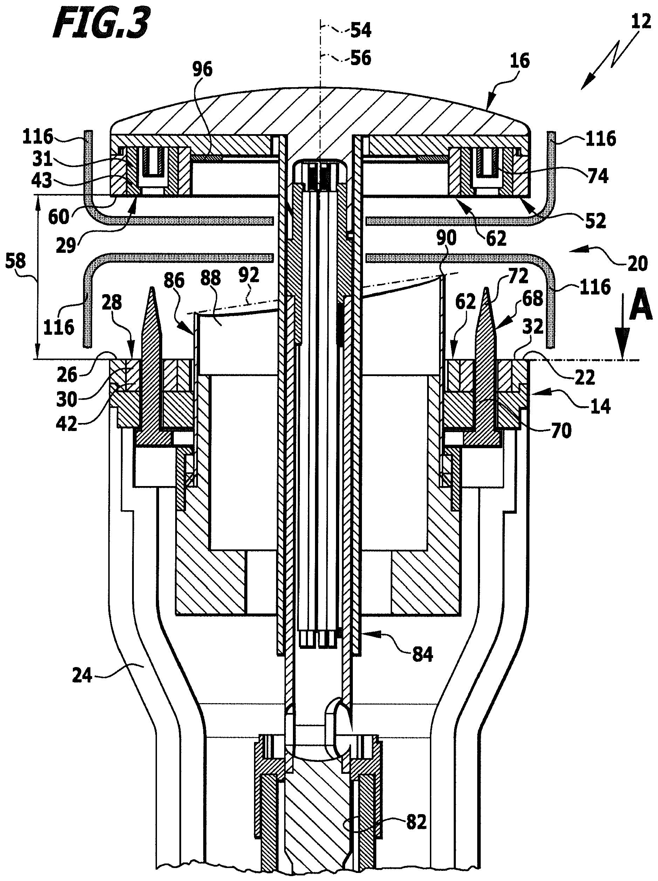

Das Kraftübertragungsglied 80 definiert an seinem distalen Ende eine sacklochförmige Aufnahme 82, in welche ein Halteglied 84 mit einem ersten freien Ende einführbar und in der Aufnahme 82 festlegbar ist. Das zweite freie Ende des im Wesentlichen stabförmigen Halteglieds 84 ist unbeweglich mit dem zweiten Werkzeugelement 16 verbunden. Auf diese Weise kann in Folge einer Verschiebung das Kraftübertragungsglied 80 in distaler Richtung das zweite Werkzeugelement 16 vom ersten Werkzeugelement 14 wegbewegt werden. Vorzugsweise ist das Instrument 12 so ausgebildet, dass das zweite Werkzeugelement 16 von einer Gewebefassstellung, wie sie schematisch in den Figuren 2 und 3 dargestellt ist und in welcher die Werkzeugelemente 14, 16 einen maximalen Abstand 58 voneinander aufweisen, in die Annäherungs- oder Gewebeverbindungsstellung bringbar ist durch Verschwenken der Betätigungsglieder 78 aufeinander zu, was eine Bewegung des Kraftübertragungsglieds 80 in proximaler Richtung zur Folge hat.

Des Weiteren umfasst das Instrument 12 eine Schneideinrichtung 86 zum Durchtrennen von Gewebe. Die Schneideinrichtung umfasst ein Schneidelement 88 mit einer in sich geschlossenen ringförmigen Schneidkante 90. Die Schneidkante 90 definiert eine relativ zur Längsachse 54 des Instruments 12 geneigte Schneidebene 92. Die Schneidebene 92 ist um circa 10° bezogen auf eine senkrecht zur Längsachse 54 verlaufende Referenzebene geneigt, die parallel zu den Werkzeugelementflächen 32 und 33 verläuft. Proximalseitig ist am Schaft 24 ein weiterer HF-Schneidanschluss 94 vorgesehen, welcher bei einer Variante des Instruments 12 elektrisch leitend mit dem Schneidelement 88 verbunden ist. Damit kann zum Beispiel eine monopolare Schneideinrichtung 86 realisiert werden, wobei zum monopolaren Schneiden am Körper des Patienten in üblicher Weise eine Neutralelektrode anzulegen wäre. Eine bipolare Schneideinrichtung 86 wird beispielsweise dadurch verwirklicht, dass der Schneidkante 90 gegenüberliegend am zweiten Werkzeugelement 16 eine Ringelektrode 96 angeordnet ist, die über eine nicht näher dargestellte elektrisch leitende Verbindung, die beispielsweise in nicht dargestellter Weise durch das Kraftübertragungsglied 80 hindurch verläuft, mit einem weiteren HF-Schneidanschluss 94 verbunden ist. Die Ringelektrode 96 selbst kann wahlweise auch segmentiert sein, beispielsweise analog den HF-Elektroden 28 und 29. Es wäre auch möglich, statt der Ringelektrode 96 die HF-Elektrode 29 als Gegenelektrode zu nutzen.

Das Schneidelement 88 ist vorzugsweise relativ zu beiden Werkzeugelementen 14, 16 verschiebbar gelagert. Die konzentrisch um die Längsachse 54 ausgebildete Schneidkante 90 kann so relativ zu den HF-Elektroden 28 und 29 verschoben werden. Zum Betätigen der Schneideinrichtung 86 ist eine Schneidbetätigungseinrichtung 98 vorgesehen mit einem vom proximalen Ende des Instruments abstehenden Betätigungsglied 100. Dieses ist über einen nicht dargestellten Mechanismus, beispielsweise ein weiteres, im Inneren des Schafts 24 verlaufendes Kraftübertragungsglied, mechanisch mit dem Schneidelement 88 gekoppelt, so dass infolge einer Bewegung des Betätigungsglieds 100 auch das Schneidelement 88 bewegt wird. Vorzugsweise ist das Betätigungsglied 100 verschiebbar und rotierbar relativ zum Schaft 24 angeordnet, so dass das Schneidelement 88 nicht nur parallel zur Längsachse 54 verschoben, sondern auch relativ zu dieser verdreht werden kann.

Um die Elektrodensegmente 30, 31 in beliebiger Weise mit einem HF-Strom beaufschlagen zu können, ist eine Steuer- und/oder Regelungseinrichtung 102 mit einer Schalteinrichtung 104 vorgesehen. Die Steuer- und/oder Regelungseinrichtung 102 ist vorzugsweise in einem Gehäuse des HF-Stromgenerators 18 angeordnet und bildet einen Teil desselben. Die Schalteinrichtung 104 ist insbesondere ausgebildet zum sequentiellen Beaufschlagen der Elektrodensegmente 30, 31 mit einem HF-Strom. Die Schalteinrichtung 104 dient insbesondere zum Ansteuern der Kontakte 66 sowie weiterer Kontakte 106, die über weitere Verbindungsleitungen 108 mit den HF-Schneidanschlüssen 94 des Instruments 12 verbindbar sind. Auf diese Weise kann mit dem HF-Stromgenerator 18 die Schneideinrichtung 86 monopolar oder bipolar betrieben werden. Zum monopolaren Betrieb wird lediglich das Schneidelement 88 mit einem HF-Strom beaufschlagt und als Gegenelektrode eine Neutralelektrode am Körper des Patienten angeordnet. Zum bipolaren Schneiden kann insbesondere eine ringförmige Gegenelektrode am zweiten Werkzeugelement 16 vorgesehen werden, zum Beispiel in Form der Ringelektrode 96, so dass dann ein HF-Strom zwischen der Gegenelektrode und dem Schneidelement 88 fließen kann. Alternativ kann auch die HF-Elektrode 29 als Gegenelektrode genutzt werden. Wird ganz auf eine Bestromung der Schneideinrichtung 86 verzichtet, so kann diese auch rein mechanisch zum Durchtrennen von Gewebe genutzt werden, und zwar mittels der vorzugsweise angeschliffenen Schneidkante 90.

Die Schalteinrichtung 104 kann ferner auch derart ausgebildet sein, dass mindestens zwei Elektrodensegmente 30, 31 einer HF-Elektrode 28, 29 gleichzeitig mit einem HF-Strom beaufschlagt werden können. Dabei ist es günstig, wenn jeweils zwischen zwei gleichzeitig mit HF-Strom beaufschlagten Elektrodensegmenten 30, 31 ein weiteres, jedoch dann unbestromtes Elektrodensegment 30, 31 angeordnet ist. Beispielsweise könnten auf diese Weise die einander gegenüberliegenden Elektrodensegmente 30 der in Figur 5 dargestellten HF-Elektrode 28 gleichzeitig bestromt werden, wobei die beiden anderen Elektrodensegmente 30 dann unbestromt blieben.

Um eine Bestromungsstärke und/oder eine Bestromungsdauer für die einzelnen Elektrodensegmente 30, 31 individuell einstellen zu können, ist die Steuer- und/oder Regelungseinrichtung 102 eine Einstelleinrichtung 110 umfassend ausgebildet. Mittels der Einstelleinrichtung 110 kann beispielsweise eine Stärke und/oder eine Frequenz des HF-Stroms eingestellt werden, ebenso wie eine Bestromungsdauer. Des Weiteren kann die Einstelleinrichtung 110 optional auch ausgebildet sein, um Bestromungssequenzen individuell einstellen zu können.

Des Weiteren umfasst die Steuer- und/oder Regelungseinrichtung 102 vorzugsweise eine Temperaturmesseinrichtung 112 zum Messen einer Elektrodensegmenttemperatur und/oder einer Gewebetemperatur. Die Temperaturmesseinrichtung 112 dient insbesondere dazu, der Steuer- und/oder Regelungseinrichtung 102 die für ein automatisches Regeln einer Bestromung der HF-Elektroden 28, 29 erforderliche Regelgröße zu liefern, nämlich eine Temperatur des Gewebes, beispielsweise indirekt über eine Temperaturmessung der Elektrodensegmente 30, 31. Beispielsweise können nicht bestromte Elektrodensegmente 30, 31 als Messkontakte zur Temperaturerfassung über eine Gewebeimpedanzmessung dienen. Auf diese Weise kann sicher gestellt werden, dass die zum Verbinden des Gewebes erforderliche Temperatur in gewünschter Weise und hochpräzise durch entsprechende Bestromung der HF-Elektroden 28, 29 erreicht wird, jedoch ein unerwünschtes Überhitzen der miteinander zu verbindenden Gewebeteile vermieden wird.

Ferner umfasst die Steuer- und/oder Regelungseinrichtung 102 optional eine Impedanzmesseinrichtung 113 zum Messen einer Gewebeimpedanz von zwischen den Werkzeugelementen 14 und 16 gehaltenem Gewebe. Die Bestimmung der Gewebeimpedanz bietet die Möglichkeit, abhängig von ihrem Wert den HF-Generator 18 zu regeln, insbesondere die von diesem bereitgestellten Parameter Spannung, Strom oder Leistung. Auf diese Weise kann die zum Verbinden der Gewebeteile in diese einzubringende Energie einfach und sicher geregelt werden. Zur Messung der Gewebeimpedanz können insbesondere die HF-Elektroden 28 und 29 genutzt werden. Eine Messung kann auch zwischen einzelnen Elektrodensegmenten 30 und 31 erfolgen, die einander gegenüberliegen. Die Gewebeimpedanzmessung kann wahlweise während der Bestromung der HF-Elektroden 28, 29 stattfinden oder wenn die HF-Elektroden 28, 29 gerade unbestromt sind. Damit kann die Veränderung des Gewebes gut und praktisch in Echtzeit überwacht und weiterer Energieeintrag dosiert, unterbunden oder gezielt weiter gestattet werden.

Mit dem oben beschriebenen chirurgischen System 10 lassen sich insbesondere schlauchförmige Gewebeteile 116 direkt miteinander verbinden, indem diese durch HF-Strom-Beaufschlagung miteinander verschweißt oder versiegelt werden. Im Einzelnen wird dabei beispielsweise folgendermaßen vorgegangen:
Zum Herstellen einer End-zu-End-Anastomose von zwei schlauchförmigen Gewebeteilen 116, wie sie beispielsweise nach einer Darmoperation, bei welcher ein Stück Darm herausgetrennt wird, erforderlich ist, werden freie Enden der Gewebeteile 116 aneinander herangeführt, so dass sie mit ihren freien Enden in Richtung auf die Längsachse weisend, ringförmig flächig aneinander anliegen, wie beispielhaft in den Figuren 3 und 4 dargestellt. Die freien Enden befinden sich dann zwischen den beiden Werkzeugelementen 14, 16, so dass die Gewebeteile 116 klemmend zwischen den Werkzeugelementen 14, 16 in der Gewebefassstellung aneinander gehalten werden können.

Die Werkzeugelemente 14, 16 werden dann aufeinander zu bewegt in die Gewebeverbindungsstellung, so dass auch die Elektrodensegmente 31 elektrisch leitend mit den HF-Anschlusskontakten 50 in der oben beschriebenen Weise verbunden sind. Zum Verschweißen der Gewebeteile 116 werden nun vorzugsweise einzelne Elektrodensegmentpaare 62 mit einem HF-Strom beaufschlagt, der dann über die zwischen den Werkzeugelementen 14, 16 gehaltenen Gewebeteilabschnitte fließt und diese erwärmt. Bei einer Temperatur von etwa 50°C bis etwa 80°C, vorzugsweise circa 65°C bis etwa 70°C erfolgt eine Veränderung in den Zellen derart, dass die Gewebeteile 116 miteinander verkleben. Das Verbindungsverfahren wird vorzugsweise derart durchgeführt, dass immer nur ein Elektrodensegmentpaar 62 gleichzeitig bestromt wird, insbesondere in einer sequentiellen Abfolge. Auf diese Weise wird eine ringförmige Verbindungslinie 114 hergestellt, welche im Wesentlichen durch die HF-Elektroden 28, 29 beziehungsweise deren Elektrodenmittellinien 44, 45 vorgegeben wird.

Dadurch, dass nicht die gesamten HF-Elektroden 28, 29 mit einem HF-Strom beaufschlagt werden, kann die Temperatur zum Verbinden der Gewebeteile 116 viel besser kontrolliert und eine Zerstörung der Zellen verhindert werden. Vorzugsweise werden die Elektrodensegmente 30, 31 nacheinander, also sequentiell bestromt, so dass die Gewebeteile 116 längs der Verbindungslinie 114 schrittweise miteinander verschweißt werden. Durch die zweireihige Anordnung der Elektrodensegmentabschnitte 38, 39, 40 und 41 wird ferner eine doppelte Verbindung zwischen den Gewebeteilen 116 hergestellt, die eine optimale Abdichtung und eine dauerhafte, stabile Verbindung der Gewebeteile 116 miteinander gewährleisten kann.

Alternativ zu einer sequentiellen Bestromung können, wie bereits oben angedeutet, auch gegenüberliegende Elektrodensegmente 30, 31 gleichzeitig bestromt werden, wodurch die Dauer zum Verbinden der Gewebeteile 116 bei dem in den Figuren 1 bis 5 schematisch dargestellten Ausführungsbeispiel halbiert werden kann.

Nach dem Verbinden der Gewebeteile 116 wird überstehendes Gewebe mittels der Schneideinrichtung 86 entfernt. Dabei wird die Schneideinrichtung 86 vorzugsweise bipolar genutzt, das heißt das Schneidelement 88 und die Ringelektrode 96 werden mit dem HF-Strom-Generator 18 verbunden und ein HF-Strom zum Durchtrennen des Gewebes über die beiden Gewebeteile 116 geleitet. Durch die geneigte Schneidkante 90 wird ein definierter Schneidfunke erzeugt, und zwar in dem Bereich, in dem der Abstand zwischen der Schneidkante 88 und der Ringelektrode 96 minimal ist. Ausgehend von diesem Bereich wandert dann der Schneidfunke automatisch entlang der Schneidkante 90 in beiden Richtungen im Kreis herum, bis das Gewebe vollständig durchtrennt ist. Die Nutzung der Schneideinrichtung 86 im bipolaren Betriebsmodus hat insbesondere den Vorteil, dass die Gewebeteile 116 beim Durchtrennen gleichzeitig auch koaguliert werden, um unerwünschte Blutungen direkt beim Durchtrennen zu stillen.

Nach dem Verbinden und Beschneiden der Gewebeteile 116 kann dann das Instrument 12 durch Zurückziehen des Schafts 24 aus dem Körper des Patienten, beispielsweise aus dessen Darm, zurückgezogen werden.

Der Schaft 24 ist, je nach Ausgestaltung des Instruments 12, vorzugweise so lang, dass beim Einsatz des Instruments 12 sowohl die Betätigungseinrichtung 76 als auch die Schneidbetätigungseinrichtung 98 noch aus dem Körper des Patienten herausragen, so dass sie von einem Operateur betätigt werden können.

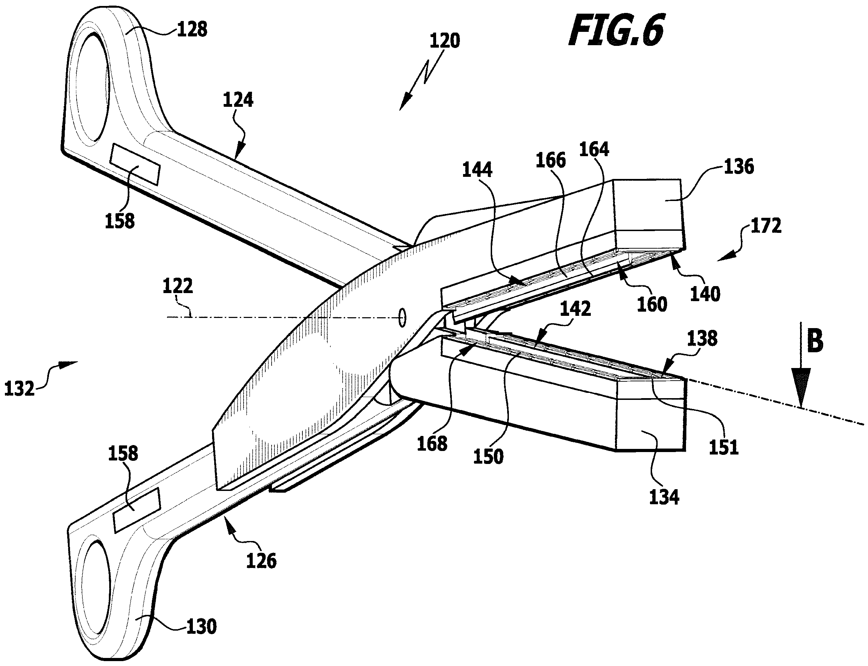

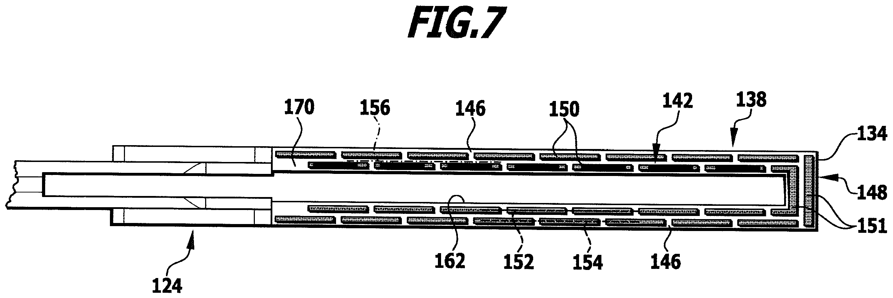

Alternativ oder zusätzlich kann das chirurgische System 10 anstelle des Instruments 12 auch ein chirurgisches Instrument beispielsweise in Form eines schematisch in den Figuren 6 und 7 dargestellten Instruments 120 umfassen. Das Instrument 120 umfasst zwei relativ zueinander um eine Schwenkachse 122 verschwenkbar aneinander gelagerte Branchen 124 und 126. An einem proximalen Ende der Branchen 124, 126 sind Fingerringe 128, 130 ausgebildet, die zusammen eine Betätigungseinrichtung 132 definieren zum Betätigen des Instruments 120.

Ausgehend von freien, distalen Enden 134 und 136 der Branchen 124 und 126 sind aufeinander zuweisend an Innenseiten derselben Werkzeugelemente 138 und 140 ausgebildet. Die Werkzeugelemente 138 und 140 sind identisch ausgebildet und liegen in einer Annäherungsstellung der Enden 134 und 136 einander gegenüber und weisen in dieser Stellung einen minimalen Abstand voneinander auf. Jedes Werkzeugelement 138, 140 umfasst eine HF-Elektrode 142, 144, die identisch und im Wesentlichen U-förmig ausgebildet sind. Jede HF-Elektrode 142, 144 umfasst zwei parallel zueinander verlaufende, sich in einer Richtung senkrecht zur Schwenkachse 122 erstreckende Elektrodenabschnitte 146 sowie einen senkrecht zu diesen verlaufenden, an den Enden 134, 136 angrenzenden Elektrodenabschnitt 148.

Der Aufbau der HF-Elektroden 142, 144 wird nachfolgend beispielhaft in Verbindung mit Figur 7 anhand der HF-Elektrode 142 näher beschrieben.

Die HF-Elektrode 142 umfasst insgesamt 30 Elektrodensegmente 150, wobei jeweils 15 Elektrodensegmente versetzt zueinander in zwei Elektrodenreihen 152, 154 parallel zueinander längs jedes Elektrodenabschnitts 146 angeordnet und gegeneinander elektrisch isoliert sind. Die Elektrodensegmente 150 sind geradlinig und streifenförmig ausgebildet. Sie definieren zwischen sich eine Elektrodenmittellinie 156, die der Form der HF-Elektrode 142 entsprechend ebenfalls U-förmig ausgebildet ist. Im Bereich des Elektrodenabschnitts 148 sind zwei weitere Elektrodensegmente 151 angeordnet, die jeweils die Elektrodenreihen 152 beziehungsweise 154 der Elektrodenabschnitte 146 vervollständigen. Die Elektrodensegmente 150 und 151 sind somit in einer von der Elektrodenmittellinie 156 definierten Richtung versetzt zueinander angeordnet.

Um die Elektrodensegmente 150, 151 mit einem HF-Strom beaufschlagen zu können, sind diese jeweils elektrisch leitend mit einem HF-Anschluss 158 in proximalen Endbereichen der Branchen 124, 126 benachbart der Fingerringe 128, 130 angeordnet. Die HF-Anschlüsse 158 können mit entsprechenden Verbindungsleitungen oder Kabeln mit dem HF-Stromgenerator 18 verbunden werden.

In der Annäherungsstellung liegen aufgrund der identischen Ausbildung der HF-Elektroden 142 und 144 gleich große oder im Wesentlichen gleich große Elektrodensegmente 150 beziehungsweise 151 einander gegenüber und weisen aufeinander zu. Sie bilden ein insgesamt mit dem Bezugszeichen 168 bezeichnetes Elektrodensegmentpaar. Insgesamt umfasst das Instrument 120 somit 32 Elektrodensegmentpaare 168.

Auch die Werkzeugelemente 138 und 140 definieren ebene Werkzeugelementflächen 170, die U-förmig ausgebildet sind. Die Elektrodensegmente 150 und 151 stehen nicht über die Werkzeugelementfläche 170 vor.

Das insgesamt zangenförmig ausgebildete Instrument 120 kann ebenfalls zum Verbinden von Gewebeteilen genutzt werden, wobei diese zwischen den Werkzeugelementen 138, 140 klemmend gehalten und dann durch entsprechende Strombeaufschlagung der Elektrodensegmente 150, 151 miteinander verschweißt oder versiegelt werden. Dazu kann, wie im Zusammenhang mit der Funktion des Instruments 12 beschrieben, eine Bestromung der Elektrodensegmente 150 sequentiell erfolgen, das heißt U-förmig umlaufend wird nach Bestromen eines Elektrodensegments 150 das nächstgelegene Elektrodensegment 150 der benachbarten Elektrodenreihe 152, 154 bestromt, bis alle Elektrodensegmente 150, 151 einmal bestromt waren. Auf diese Weise kann eine zweireihige Verbindungslinie zum Verbinden zweier Gewebeteile hergestellt werden. Alternativ ist auch beim Instrument 120 eine gleichzeitige Bestromung von zwei oder auch mehr Elektrodensegmenten 150, 151 denkbar, wobei vorzugsweise aneinander angrenzende Elektrodensegmente 150, 151 nicht gleichzeitig bestromt werden, sondern bevorzugt mindestens eines, besser zwei oder drei Elektrodensegmente 150, 151 zwischen gleichzeitig bestromten Elektrodensegmenten 150, 151 unbestromt bleiben.

Das Instrument 120 kann optional auch eine Schneideinrichtung 160 umfassen, wie sie schematisch in Figur 6 dargestellt ist. Zwischen den Elektrodenabschnitten 146 ist jeweils ein Schlitz 162 an den Werkzeugelementen 138, 140 ausgebildet. Im Schlitz 162 an der Branche 126 ist ein Schneidelement 164 mit einer in Richtung auf den Schlitz 162 der Branche 124 weisenden Schneidkante 166 gehalten und optional relativ zum Werkzeugelement 136 bewegbar. Damit kann zum Beispiel bereits beim Schließen der Branchen 124 und 126 das zwischen den Werkzeugelementen 138 und 140 gehaltene Gewebe durchtrennt werden. Optional kann das Schneidelement 164 auch monopolar oder bipolar genutzt werden, wobei beispielsweise die HF-Elektrode 142 als Gegenelektrode zum Schneidelement 164 bei einem biopolaren Einsatz genutzt werden kann. Zum monopolaren Betrieb wird lediglich das Schneidelement 164 mit einem HF-Strom beaufschlagt und als Gegenelektrode eine Neutralelektrode am Körper des Patienten angeordnet. In beiden Fällen ist das Schneidelement 164 vorzugsweise auch elektrisch leitend mit einem Kontakt der HF-Anschlüsse 158 verbunden.

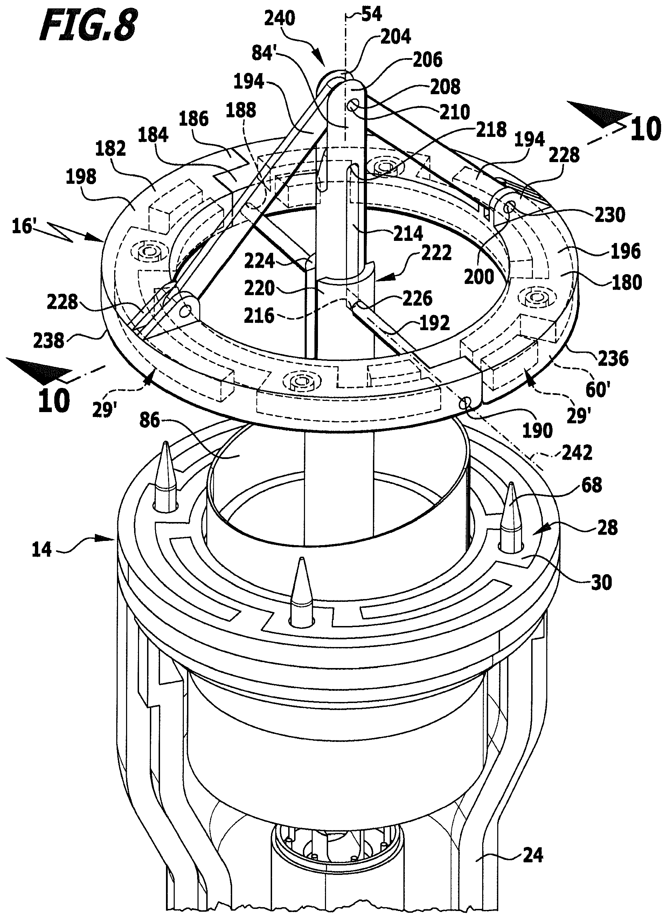

In den Figuren 8 bis 11 ist eine Variante des Instruments 12 dargestellt, das sich durch die Ausgestaltung des zweiten Werkzeugelements unterscheidet, das in den Figuren 8 bis 11 mit dem Bezugszeichen 16' bezeichnet ist. Das Werkzeugelement 16' nimmt in einer Betriebsstellung, in welcher es in die oben beschriebene Annäherungsstellung bringbar ist, eine kreisringfähige Form ein. Es umfasst zwei Kreisringabschnitte 180 und 182, die sich jeweils über einen auf die Längsachse 54 bezogenen Winkel von etwa 180° erstrecken. Freie Enden der Kreisringabschnitte 180, 182 sind nur halb so breit wie die Kreisringabschnitte 180, 182 im übrigen Bereich und dienen als Lagerböcke 184 und 186. Die Lagerböcke 184 und 186 sind jeweils mit einer Querbohrung 188 und 190 versehen, in die ein zylindrischer Stab 192 eingesetzt ist. Die Lagerböcke 184 liegen an den Lagerböcken 186 auf deren der Längsachse 54 zugewandten Seite an. Der Stab 192 ist drehfest in den Querbohrungen 190 des Kreisringabschnitts 182 festgelegt. Die Querbohrung 188 ist in ihrem Innendurchmesser so bemessen, dass der Kreisringabschnitt 180 relativ zum Stab 192 um eine von diesem definierte Schwenkachse 242 und damit relativ zum Kreisringabschnitt 182 verschwenkbar ist.

Die beiden Kreisringabschnitte 180 und 182 sind jeweils zusätzlich über einen stabförmigen Lenker 194 mit einem Halteglied 84', welches eine mit der Längsachse 54 zusammen fallende Haltegliedlängsachse definiert, gekoppelt. Das Halteglied 84' ist, analog wie das Halteglied 84, mit dem Kraftübertragungsglied 80 gekoppelt beziehungsweise koppelbar, und auf diese Weise relativ zum Schaft 24 in distaler und proximaler Richtung bewegbar. Zum beweglichen Anlenken der Lenker 194 am Halteglied 84' ist letzteres im Bereich seines distalen Endes mit einem Schlitz 204 versehen, welcher sich quer zu einer vom Stab 192 definierten Längsachse erstreckt. Auf diese Weise werden zwei Schenkel 206 ausgebildet, die mit einer fluchtenden Querbohrung 208 versehen sind, in welche ein zylindrischer Lagerstift 210 drehfest eingesetzt ist. Die Lenker 194 sind an ihren ersten Enden mit einer Aufnahmebohrung 212 versehen, durch die sich der Lagerstift 210 erstreckt und die einen Innendurchmesser aufweist, um eine Verschwenkbewegung der Lenker 194 um eine vom Lagerstift 210 definierte Schwenkachse zu gestatten.

Etwas proximalseitig des Schlitzes 204 erstreckt sich im Halteglied 84' weiter in proximaler Richtung ein Längsschlitz oder Langloch 214, welches vom Stab 192 durchsetzt ist. Auf diese Weise ist der Stab 192 definiert und parallel zu sich selbst in einer Richtung parallel zur Längsachse 54 verschiebbar. Ein proximales Ende des Langlochs 214 bildet einen proximalen Endanschlag für den Stab 192, ein distales Ende 218 des Langlochs 214 einen distalen Endanschlag für den Stab 192.

Zum Bewegen des Stabes 192 dient ein Betätigungsmechanismus 222, welcher ein hülsenförmiges Kraftübertragungselement 220 umfasst, dessen Innendurchmesser an den Außendurchmesser des Halteglieds 84' angepasst und somit auf dem Halteglied 84' in distaler und proximaler Richtung verschiebbar ist. Das Kraftübertragungselement 220 ist benachbart seines distalen Endes 224 mit einer Bohrung 226 versehen, die der Stab 192 durchsetzt. Der Stab 192 ist relativ zur Bohrung 226 rotierbar. Der Betätigungsmechanismus 222 kann ferner einen Teil des oben beschriebenen Betätigungsmechanismus 76 bilden. Dies bedeutet, dass eine Bewegung des Stabes 192 beispielsweise auch durch eine Verschwenkung der Betätigungsglieder 100 relativ zueinander möglich ist. Alternativ wäre es denkbar, eine weitere Betätigungseinrichtung analog der Betätigungsmechanismus 76 vorzusehen, die ein oder zwei weitere Betätigungsglieder, ähnlich den Betätigungsgliedern 100, umfasst, um gezielt eine Relativbewegung zwischen dem Kraftübertragungselement 220 und dem Halteglied 84' zu realisieren.

Auf Oberseiten der Kreisringabschnitte 180 und 182 sind jeweils parallel zueinander zwei Lagerböcke 228 angeordnet, die parallel zur Querbohrung 208 mit Bohrungen 230 versehen sind. Zwischen den Lagerböcken 228 ist jeweils ein weiteres freies Ende der Lenker 194 auf der in den Bohrungen 230 eingesetzten Lagerwelle 200 verschwenkbar gelagert. Durch die beschriebene Anordnung der Lenker 194, die auch als Anlenkglieder bezeichnet werden können, ist sichergestellt, dass sie mit einem Ende am zweiten Werkzeugelement 16' an einem Angriffs- oder Gelenkpunkt angreifen, welcher von der Schwenkachse 242 beabstandet ist.

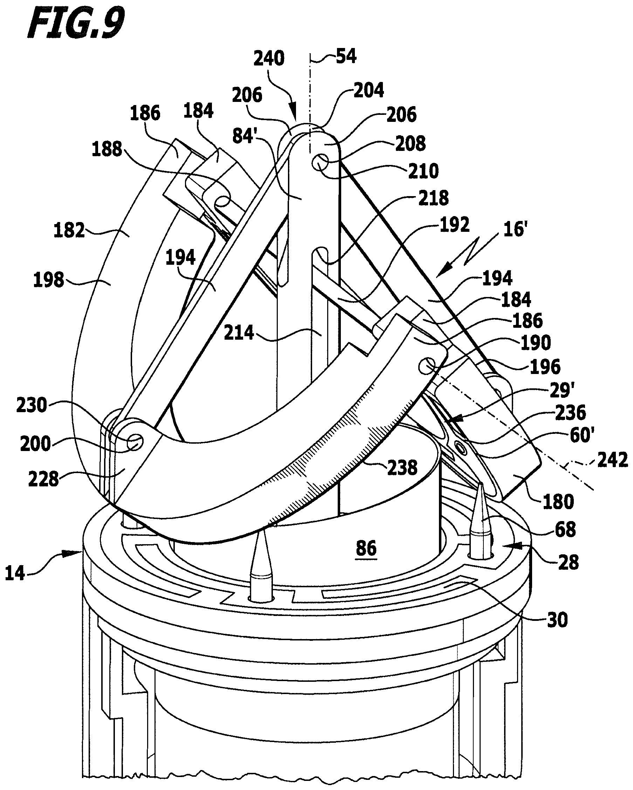

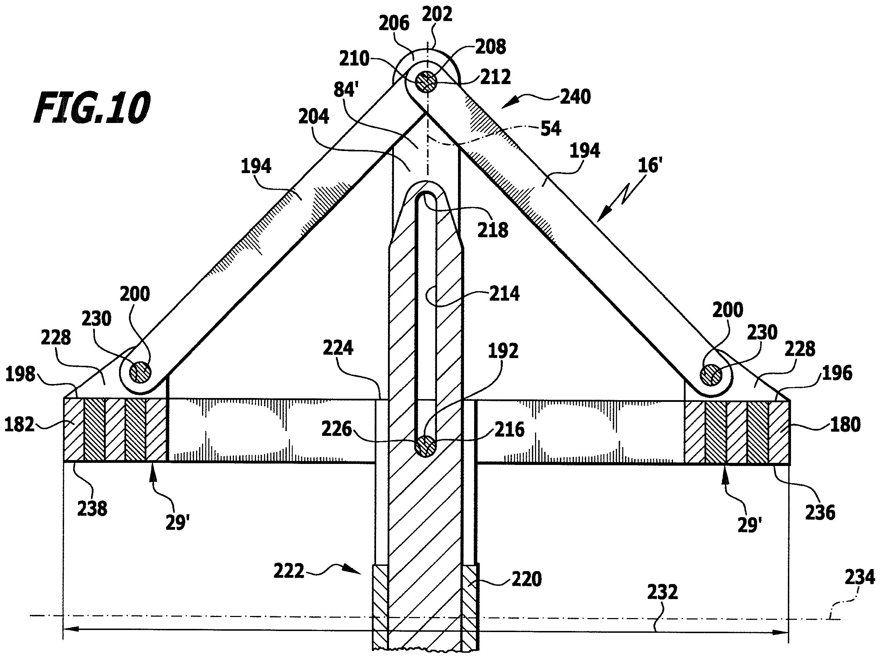

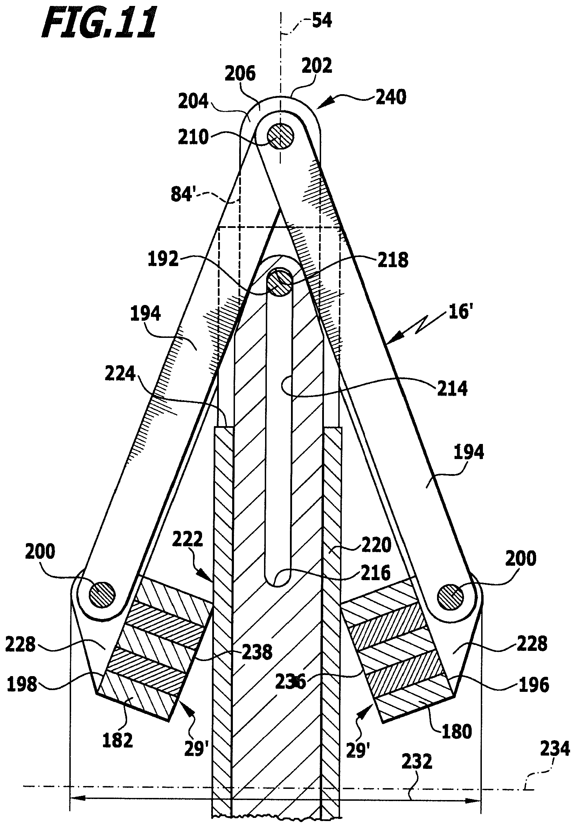

Mittels des Betätigungsmechanismus 222 kann das zweite Werkzeugelement 16' von der bereits erwähnten Betriebsstellung, die schematisch in den Figuren 8 und 10 dargestellt ist, in die Entfernungsstellung gebracht werden, die beispielhaft in Figur 11 dargestellt ist. Figur 9 stellt eine Zwischenstellung schematisch dar, also eine Stellung, zwischen der Betriebsstellung und der Entfernungsstellung. Wie durch Vergleich der beiden Figuren 10 und 11 leicht erkennbar, ist ein Flächenbereich einer senkrechten Projektion des zweiten Werkzeugelements 16' auf eine Projektionsebene 234, welche senkrecht zur Längsachse 54, also zur Schaftrichtung im Bereich des zweiten Werkzeugelements 16', verläuft, in der Entfernungsstellung kleiner ist als in der Betriebsstellung. Dies wird durch eine Bewegung des hülsenförmigen Kraftübertragungselements 220 ausgehend von der Betriebsstellung erreicht, in der der Stab 192 am proximalen Ende 216 anschlägt und sich Unterseiten 236 und 238 der Kreisringabschnitte 180 und 182 parallel zur Projektionsebene 234 erstrecken. Bewegt man das Kraftübertragungselement 220 in distaler Richtung, wird der Stab 192 im Langloch 214 in distaler Richtung zwangsgeführt. Durch die gelenkige Verbindung der Kreisringabschnitte 180 und 182 relativ zueinander und über die beiden Lenker 194 mit dem Halteglied 84' schwenken die Kreisringabschnitte 180 und 182 um die Schwenkachse 242 in Richtung auf die Längsachse 54 hin. Das zweite Werkzeugelement 16' wird auf diese Weise zusammengefaltet oder zusammengeklappt. Es ist also durch die gelenkige Anordnung der Kreisringabschnitte 180 und 182 mittels der Lenker 194 ein Faltmechanismus 240 ausgebildet zum Überführen des zweiten Werkzeugelements 16' von der Betriebsstellung in die Entfernungsstellung.

Bisher nicht erwähnt ist die Ausgestaltung der Unterseiten 236 und 238 des zweiten Werkzeugelements. Diese können entweder eine einzige, im Wesentlichen durchgehende Ringelektrode aufweisen, die eine einzige Gegenelektrode zur HF-Elektrode 28 des ersten Werkzeugelements 14 bildet. Alternativ kann auf den Unterseiten 236 und 238 analog der HF-Elektrode 29 auch eine HF-Elektrode mit zwei oder mehr Elektrodensegmenten 31 ausgebildet sein, vorzugsweise entsprechend der HF-Elektrode 29. Dies gestattet dann in der Betriebsstellung ein Verbinden von Gewebeteilen 116 in der oben beschriebenen Weise.

Nach dem Verbinden der Gewebeteile kann dann der Faltmechanismus 240 betätigt werden, beispielsweise durch entsprechende Betätigung des beschriebenen Betätigungsmechanismus 222, wodurch das Halteglied 84' in distaler Richtung bewegt wird. Ist das Kraftübertragungselement 220 beispielsweise unbeweglich relativ zum Schaft 24 angeordnet, dann kann bei einer Bewegung in distaler Richtung des Kraftübertragungsglieds 80 automatisch das zweite Werkzeugelement 16' zusammengeklappt werden. Durch den in der Entfernungsstellung deutlich herabgesetzten Flächenbedarf kann das zweite Werkzeugelement durch eine durch das Verbinden der Gewebeteile 116 ausgebildete Verbindungsstelle hindurchgeführt werden beim Entfernen des Instruments 12, und zwar ohne die Verbindungsstelle zu dehnen, was deutlich schonender ist, als das zweite Werkzeugelement in der Betriebsstellung durch die Verbindungsstelle hindurchzuführen.

Es versteht sich von selbst, dass elektrisch leitende Verbindungen von der Elektrode 29 zu den HF-Anschlusskontakten 50 zum Beispiel über die Lenker 94 und das Halteglied 84' zu den HF-Anschlusskontakten 50 im proximalen Endbereich des Schafts 24 geführt werden können.

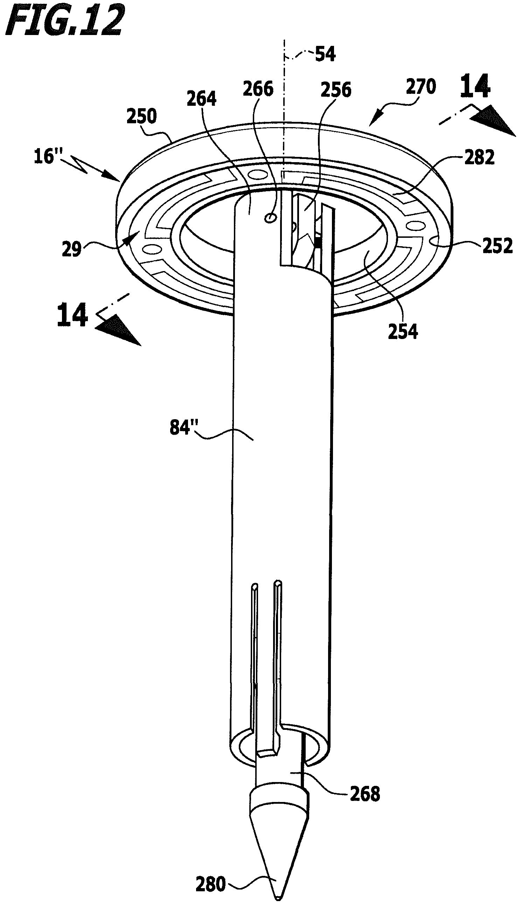

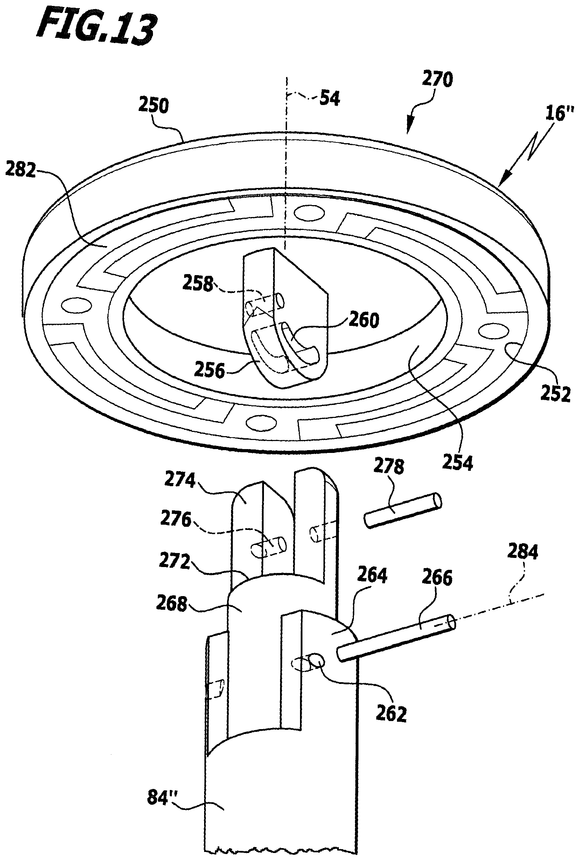

Eine weitere Variante eines zweiten Werkzeugelements ist in den Figuren 12 bis 15 insgesamt mit dem Bezugszeichen 16" bezeichnet. Es ersetzt beispielsweise die oben beschriebenen Werkzeugelemente 16 und 16' des Instruments 12.

Das zweite Werkzeugelement 16" ist im Wesentlichen tellerförmig ausgebildet mit einer in distaler Richtung weisenden, schwach konvex gekrümmten Außenseite 250.

Auf einer Unterseite des zweiten Werkzeugelements 16" ist eine Ringnut 252 ausgebildet, die in proximaler Richtung weisend geöffnet ist. Zentralmittig ist eine im Wesentlichen kreisförmige Vertiefung 254 ausgebildet, in der ein im Wesentlichen quaderförmiger Lagervorsprung 256 angeordnet ist, welcher koaxial zur Längsachse 54 in proximaler Richtung von der Unterseite des zweiten Werkzeugelements 16" abstehend ausgebildet ist. Der Lagervorsprung 256 ist mit einer Querbohrung 258 versehen, welche bezogen auf die Längsachse 54 windschief verläuft. Des Weiteren ist am Lagervorsprung 256 ein gekrümmter Führungsschlitz 260 ausgebildet, welcher in proximaler Richtung weisend konvex gekrümmt ist. Ein proximales Ende des Lagervorsprungs 256 weist eine abgerundete Außenkontur auf.

Das zweite Werkzeugelement 16" ist verschwenkbar an einem hülsenförmigen Halteglied 84" gelagert. Zu diesem Zweck ist das Halteglied 84" mit einer Querbohrung 262 versehen, die eine Wand 264 des Halteglieds 84" an zwei Stellen durchsetzt. In die Querbohrung 262 ist ein Lagerstift 266 drehfest eingesetzt. Er durchsetzt gleichzeitig die Querbohrung 258 derart, dass der Lagervorsprung 256 um eine vom Lagerstift 266 definierte Schwenkachse 284 verschwenkbar ist. Um einen auch beim zweiten Werkzeugelement 16" vorgesehenen Faltmechanismus 270 betätigen zu können, ist ein Kraftübertragungselement 268 vorgesehen, welches im Wesentlichen stabförmig ausgebildet ist und das Halteglied 84" koaxial zur Längsachse 54 durchsetzt. Von einer distalseitigen Endfläche 272 des Kraftübertragungselements 268 sind zwei Lagerschenkel 274 parallel zueinander und in distaler Richtung weisend abstehend angeordnet, welche jeweils von einer fluchtenden Bohrung 276 durchsetzt sind. In die Bohrungen 276 drehfest eingesetzt ist ein weiterer Lagerstift 278, welcher parallel zum Lagerstift 266 orientiert ist. Ein Außendurchmesser des Lagerstifts 278 ist so bemessen, dass er den Führungsschlitz 260 durchsetzen und relativ zu diesem bewegt werden kann.

Ein proximales Ende 280 des Kraftübertragungselements 268 ist vorzugsweise mit dem Kraftübertragungsglied 80 koppelbar, so dass sich in Folge einer Bewegung desselben auch das zweite Werkzeugelement 16" bewegen lässt.

In die Ringnut 252 eingesetzt ist ein ringförmiges Elektrodenelement 282, welches vorzugsweise eine HF-Elektrode 29 in der oben beschriebenen Form umfasst, die in den Figuren 12 bis 15 der Übersichtlichkeit wegen nicht im Einzelnen dargestellt ist. Alternativ kann auch eine einfache, durchgehende Ringelektrode am Elektrodenelement 282 ausgebildet sein.

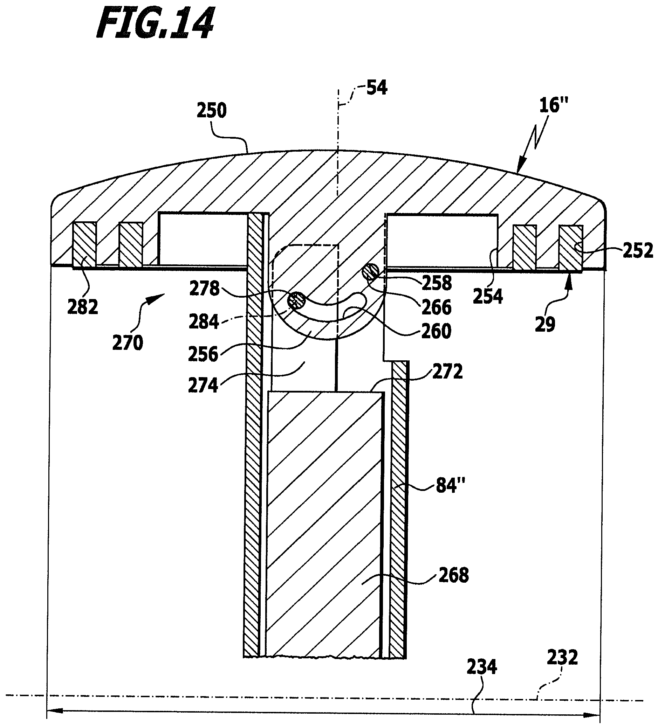

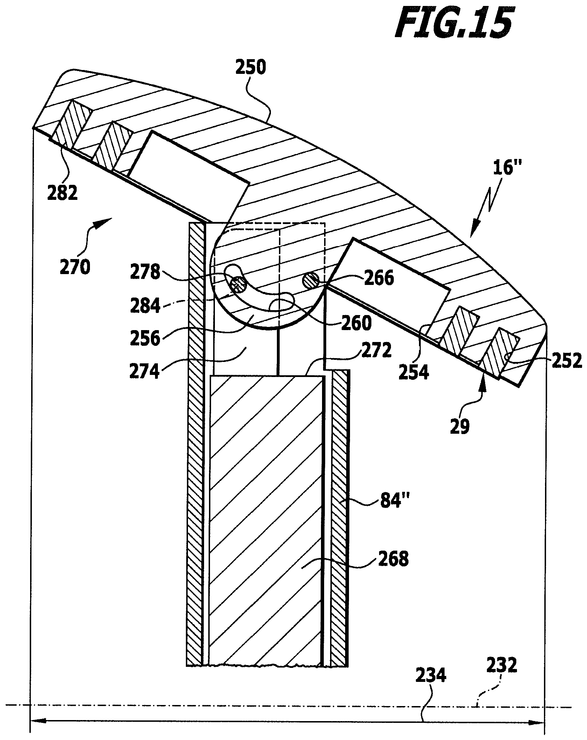

Zum Überführen des zweiten Werkzeugelements 16" von der Betriebsstellung in die Entfernungsstellung wird das Kraftübertragungselement 268 in distaler Richtung bewegt. Durch den speziell gekrümmten Führungsschlitz 260 wird der Lagerstift 278 in diesem zwangsgeführt und bewirkt somit eine zwangsgeführte Verschenkung des zweiten Werkzeugelements 16" um die Schwenkachse 284. Im Wesentlichen kann so das zweite Werkzeugelement 16" um nahezu 90° verschwenkt werden, so dass auch bei dieser Variante des Werkzeugelements 16" eine senkrechte Projektion 232 desselben auf die Projektionsebene 234 in der Entfernungsstellung kleiner ist als in der Betriebsstellung, wie dies schematisch in den Figuren 14 und 15 dargestellt ist. Auf diese Weise wird in der Entfernungsstellung beim Entfernen des Instruments 12 eine Überdehnung der Verbindungsstelle zwischen den miteinander verbundenen Gewebeteilen 116 vermieden.

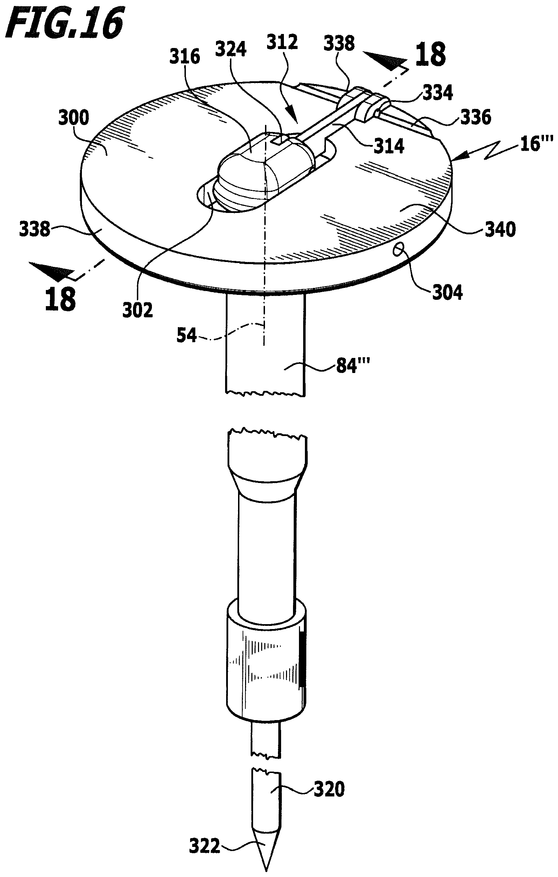

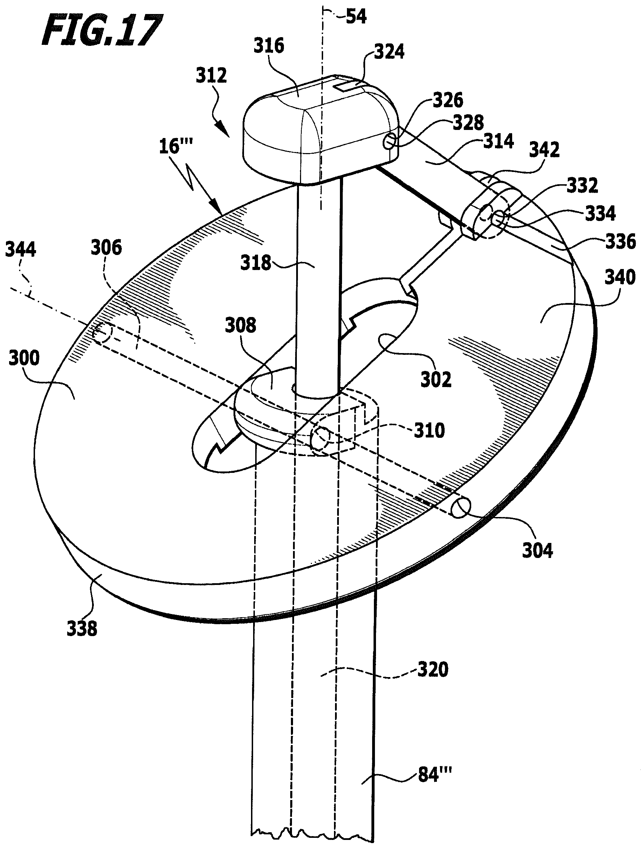

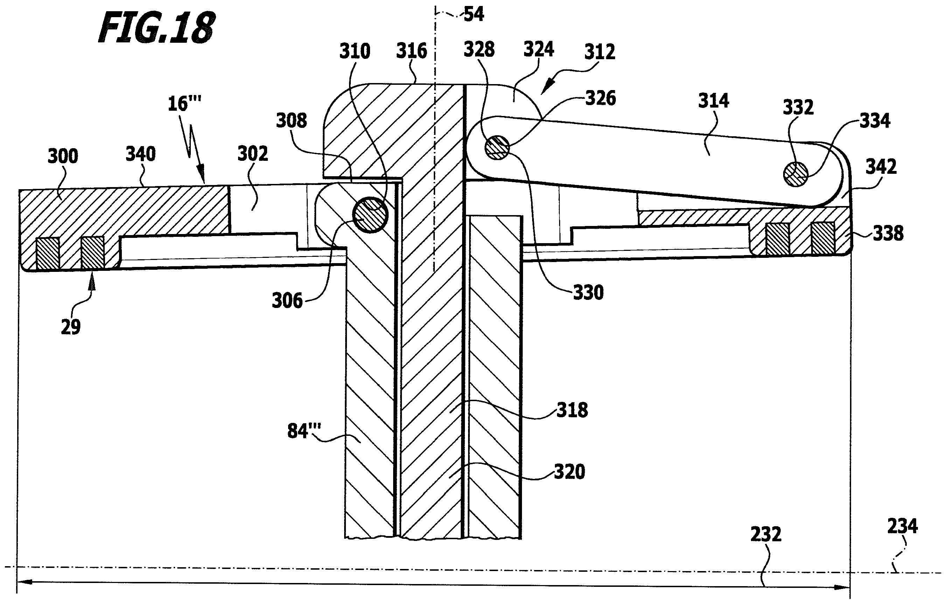

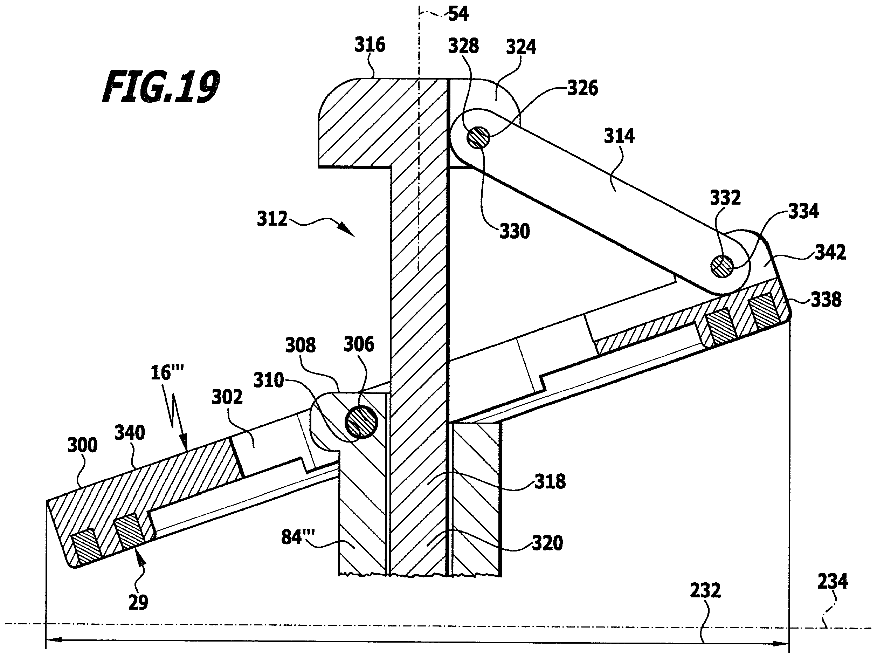

Eine weitere Ausführungsform eines insgesamt mit dem Bezugszeichen 16"' versehenen zweiten Werkzeugelements ist in den Figuren 16 bis 19 dargestellt. Es kann beim Instrument 12 anstelle der bisher beschriebenen zweiten Werkzeugelemente 16, 16' und 16" genutzt werden.

Das zweite Werkzeugelement 16'" ist im Wesentlichen tellerförmig ausgebildet und umfasst eine Scheibe 300. Diese ist in ihrem Zentrum mit einem quer verlaufenden, länglich ovalen Schlitz 302 versehen. Eine Bohrung 304 durchsetzt die Scheibe 300 etwas seitlich versetzt zu deren Mittelpunkt, der im Bereich des Schlitzes 302 liegt. In die Bohrung 304 drehfest eingesetzt ist ein Lagerstift 306, der den Schlitz 302 ebenfalls durchsetzt. In den Bereich des Schlitzes 302 hinein ragt ein distales Ende eines Halteglieds 84"', welches hülsenförmig ausgebildet ist. Das Halteglied 84'" ist proximalseitig von seinem Ende 308 mit einer Bohrung 310 versehen, deren Innendurchmesser an den Außendurchmesser des Lagerstifts 306 derart angepasst ist, dass der Lagerstift 306 relativ zur Bohrung 310 in dieser rotierbar ist. Dies ermöglicht dann insgesamt eine Verschwenkung der Scheibe 300 um eine vom Lagerstift 306 definierte Längsachse.

Zum zwangsbetätigten Verschwenken der Scheibe 300 dient ein Faltmechanismus 312, welcher die Scheibe 300 über einen Lenker 314 gelenkig mit einem distalen Ende 316 eines Kraftübertragungselements 318 koppelt. Das Kraftübertragungselement 318 weist einen langgestreckten, stabförmigen Abschnitt 320 auf, dessen proximales Ende 322 mit dem Kraftübertragungsglied 80 koppelbar ist. Das Ende 316 ist gegenüber dem Abschnitt 320 kopfförmig verdickt und nahezu quaderförmig geformt. An einer Seite desselben ist ein seitlich geöffneter Schlitz 324 ausgebildet. Ferner ist eine Querbohrung 326 vorgesehen, die den Schlitz 324 quer durchsetzt. In die Querbohrung 326 drehfest eingesetzt ist ein Lagerstift 328. Der stabförmige Lenker 314 ist ebenfalls mit einer Bohrung 330 versehen und auf dem Lagerstift 328 verschwenkbar gelagert. Benachbart eines gegenüber liegenden Endes des Lenkers 314 ist eine weitere Bohrung 332 vorgesehen. Sie dient zur Lagerung des Lenkers 314 auf einem weiteren Lagerstift 334. Dieser ist in einer weiteren Bohrung 336 der Scheibe 300 eingesetzt. Die Bohrung 336 ist parallel zur Bohrung 304 orientiert und außerhalb des Schlitzes 302 benachbart eines Randes 338 der Scheibe 300 angeordnet, und zwar der Bohrung 304 bezogen auf die Längsachse 54 gegenüberliegend. Auf einer Oberseite 340 der Scheibe 300 ist ausgehend vom Rand 338 eine Nut 342 eingearbeitet, in die das Ende des Lenkers 314 mit dessen Bohrung 332 eintaucht. Auf diese Weise ist der Lenker 314 gelenkig auf dem Lagerstift 334 gelagert. Damit greift der Lenker 314 mit einem Ende am zweiten Werkzeugelement 16'" an einem Angriffs- oder Gelenkpunkt an, welcher von einer von der Längsachse des Lagerstifts 306 definierten Schwenkachse 344 beabstandet ist.

Der Faltmechanismus 312 wird betätigt, indem das Kraftübertragungselement 318 in distaler Richtung bewegt wird. Dies hat zur Folge, dass der Lenker 314 relativ zur Scheibe 300 abgewinkelt wird. Je weiter das Kraftübertragungsglied 318 in distaler Richtung bewegt wird, umso weiter zieht dabei der Lenker 314 den Bereich der Scheibe 300 in distaler Richtung, an welchem die Nut 342 vorgesehen ist. In einer extremen Stellung ist dann die Scheibe 300 nahezu parallel zur Längsachse 54 ausgerichtet. Insgesamt ist es somit auch beim zweiten Werkzeugelement 16"' möglich, eine Entfernungsstellung so zu realisieren, in welcher eine senkrechte Projektion 232 desselben auf die Projektionsebene 234, welche senkrecht zur Längsachse 54 verläuft, kleiner ist als in der Betriebsstellung.

Am zweiten Werkzeugelement 16'" kann ebenfalls eine HF-Elektrode 29 in einer Form wie oben beim zweiten Werkzeugelement 16 beschrieben angeordnet oder ausgebildet sein. Alternativ ist es auch denkbar, eine in sich geschlossene, ringförmige Elektrode vorzusehen, die nicht in Elektrodensegmente unterteilt ist. Ähnlich wie das zweite Werkzeugelement 16" das Elektrodenelement 282 umfasst, können bei den zweiten Werkzeugelementen 16' und 16'" ebenfalls Elektrodenelemente vorgesehen sein, beispielsweise in Form des Elektrodenelements 282 oder aber auch des Elektrodenelements 52.

Wie bereits oben im Zusammenhang mit dem zweiten Werkzeugelement 16' erwähnt, können die an den zweiten Werkzeugelementen 16" und 16"' vorgesehenen HF-Elektroden in üblicher Weise durch Vorsehen entsprechender elektrisch leitender Verbindungen am Instrument 12 mit den HF-Anschlusskontakten 50 verbunden werden.

Alle oben beschriebenen ersten und zweiten Werkzeugelemente 14, 16, 16', 16", 16'" sowie 138 und 140 sind vorzugsweise aus entweder elektrisch leitenden oder elektrisch isolierenden Bauteilen aufgebaut. Denkbar sind auch Bauteile, die teilweise elektrisch leitend und teilweise elektrisch isolierend sind. Die Bauteile selbst können insbesondere komplett aus elektrisch leitenden oder elektrisch isolierenden Materialien hergestellt sein, wobei die elektrisch isolierenden Bauteile auch aus einem elektrisch leitenden Material hergestellt sein können, das insbesondere mit einer elektrisch isolierenden äußeren Hülle oder Beschichtung versehen ist. Als elektrisch isolierende oder nicht leitende Materialien können insbesondere Kunststoffe verwendet werden, welche bei den im Einsatz des chirurgischen Systems 10 auftretenden Temperaturen noch eine ausreichende Festigkeit aufweisen. Es eignen sich zum Beispiel sowohl Thermoplaste als auch Duroplaste. Alternativ kann als Isoliermaterial auch keramisches Material zum Einsatz kommen. Insbesondere können die Bauteile der Werkzeugelemente 14, 16, 16', 16", 16"' sowie 138 und 140 aus einer Keramik hergestellt sein. Eine Keramik zu verwenden hat insbesondere gegenüber vielen Kunststoffen den Vorteil, dass sie auch bei sehr hohen Temperaturen eine ausreichende Stabilität aufweist. Die HF-Elektroden 28 und 29 sind vorzugsweise aus einem Metall oder einer Metalllegierung hergestellt. Alternativ ist auch der Einsatz von elektrisch leitenden Keramiken zur Ausbildung der HF-Elektroden 28 und 29 denkbar, sofern sie die Anforderungen der Anwendung von HF-Strom erfüllen.

Die Werkzeugelemente 14, 16, 16', 16", 16'" sowie 138 und 140 können beispielsweise wie nachfolgend beschrieben hergestellt werden. Die einzelnen Teile, Bauteile oder Komponenten der Werkzeugelemente 14, 16, 16', 16", 16"' sowie 138 und 140 können insbesondere separat hergestellt und anschließend zusammengefügt werden, zum Beispiel durch Verkleben. Alternativ ist es zum Beispiel auch möglich, die elektrisch leitenden Teile der HF-Elektroden 28 und 29 als Einlegeteile in ein Kunststoffspritzwerkzeug einzulegen und mit einem Kunststoff zu umspritzen. Wie bereits erwähnt, können die Elektroden aus einem Metall oder einer elektrisch leitenden Keramik ausgebildet sein. Bei einer Segmentierung der HF-Elektroden 28 und 29 wie oben beschrieben, muss dann beispielsweise eine entsprechende Anzahl elektrisch leitender Elektrodensegmente aus einem Metall oder einer Metalllegierung oder einer elektrisch leitenden Keramik in das Kunststoffspritzwerkzeug vor Umspritzen mit einem geeigneten Kunststoff eingelegt werden.

Bei einer rein keramischen Ausführung der Werkzeugelemente 14, 16, 16', 16", 16'" sowie 138 und 140 bietet sich insbesondere ein Keramikpulverspritzgussverfahren an, beispielsweise die sogenannte "2K CIM"-Technologie, ein zwei Komponenten-Mikro-Keramikpulverspritzgussverfahren. Dabei werden zwei verschiedene Keramiken in einem Spritzprozess abgespritzt, die bei den fertigen Werkzeugelementen 14, 16, 16', 16", 16'" sowie 138 und 140 die elektrisch leitenden sowie elektrisch isolierenden Anteile ausbilden. Nach dem Abspritzen werden die zwei verschiedenen Keramiken zusammen gesintert. Dabei kann es sich zum Beispiel um eine Al2O3 Keramik und um eine Mischkeramik aus Al2O3 und TiN handeln.

## Patentansprüche

1. Chirurgisches System (10) zum Verbinden von schlauchförmigen Körpergewebeteilen bei einer End-zu-End-, Seit-zu-End- oder Seit-zu-Seit-Anastomose, umfassend
ein chirurgisches Instrument (12) mit einem ersten ring- oder tellerförmig ausgebildeten Werkzeugelement (14) und einem zweiten ring- oder tellerförmig ausgebildeten Werkzeugelement (16'; 16"; 16"'), welche relativ zueinander bewegbar sind und jeweils eine ringförmige, ein- oder mehrteilig ausgebildete, in sich geschlossene HF-Elektrode (28, 29) umfassen, die in einer Annäherungsstellung der Werkzeugelemente (14, 16'; 16"; 16'") einen minimalen Abstand (58) voneinander definieren, einander gegenüberliegen und aufeinander zu weisen, wobei
das Instrument (12) einen Schaft (24) aufweist, an dessen distalem Ende das erste Werkzeugelement (14) angeordnet oder ausgebildet ist,
**dadurch gekennzeichnet, dass**
das zweite Werkzeugelement (16'; 16"; 16'") von einer Betriebsstellung, in welcher es in die Annäherungsstellung bringbar ist, in eine Entfernungsstellung und/oder umgekehrt bringbar ist, und
in der Entfernungsstellung ein Flächenbereich einer senkrechten Projektion (232) des zweiten Werkzeugelementes (16') auf eine Projektionsebene (234), welche senkrecht zur Schaftrichtung (54) im Bereich des zweiten Werkzeugelements (16') verläuft, kleiner ist als in der Betriebsstellung.

2. Chirurgisches System nach Anspruch 1, **dadurch gekennzeichnet, dass** das zweite Werkzeugelement (16'; 16"; 16"') an einem Halteglied (84'; 84"; 84"') bewegbar gelagert ist.

3. Chirurgisches System nach Anspruch 2, **dadurch gekennzeichnet, dass** das zweite Werkzeugelement (16'; 16"; 16"') um eine Schwenkachse (242; 284; 344) verschwenkbar gelagert ist, welche quer, insbesondere senkrecht, zu einer vom Halteglied (84'; 84"; 84"') definierten Haltegliedlängsachse (54) verläuft.

4. Chirurgisches System nach Anspruch 2 oder 3, **gekennzeichnet durch** einen Faltmechanismus (240; 270; 312) zum Überführen des zweiten Werkzeugelements (16'; 16"; 16"') von der Betriebsstellung in die Entfernungsstellung.

5. Chirurgisches System nach Anspruch 4, **dadurch gekennzeichnet, dass** der Faltmechanismus (240; 270; 312) ein Kraftübertragungselement (220; 268; 318) zum Übertragen einer Betätigungskraft auf das zweite Werkzeugelement (16'; 16"; 16'") zum Überführen desselben von der Betriebsstellung in die Entfernungsstellung und/oder umgekehrt umfasst.

6. Chirurgisches System nach Anspruch 5, **dadurch gekennzeichnet, dass** das Kraftübertragungselement (220; 268; 318) und das Halteglied (84'; 84"; 84"') relativ zueinander verschieb- und/oder verdreh- und/oder verschraubbar ausgebildet sind.

7. Chirurgisches System nach Anspruch 5 oder 6, **gekennzeichnet durch** einen mit dem Faltmechanismus (240; 270; 318) und/oder dem Kraftübertragungselement (220; 268; 318) und/oder dem Halteglied (84'; 84"; 84"') gekoppelten Betätigungsmechanismus (76) zum Betätigen des Faltmechanismus (240; 270; 318) und/oder zum Bewegen des Kraftübertragungselements (220; 268; 318) und/oder des Halteglieds (84'; 84"; 84'") relativ zum Schaft.

8. Chirurgisches System nach einem der Ansprüche 5 bis 7, **dadurch gekennzeichnet, dass** das zweite Werkzeugelement (16'; 16"') und das Kraftübertragungselement (220; 318) über mindestens ein Anlenkglied (194; 314) miteinander gelenkig gekoppelt sind.

9. Chirurgisches System nach einem der voranstehenden Ansprüche, **dadurch gekennzeichnet, dass** das zweite Werkzeugelement (16') zwei- oder mehrteilig ausgebildet ist

10. Chirurgisches System nach einem der voranstehenden Ansprüche, **dadurch gekennzeichnet, dass** das zweite Werkzeugelement (16') mindestens zwei Werkzeugelementteile (180, 182) umfasst, welche relativ zueinander beim Übergang von der Betriebsstellung in die Entfernungsstellung bewegbar sind.

11. Chirurgisches System nach einem der voranstehenden Ansprüche, **dadurch gekennzeichnet, dass** das zweite Werkzeugelement (16'; 16"; 16"') ein in Schaftrichtung (54) bewegbares und in Richtung auf das erste Werkzeugelement (14) hin- und von diesem weg bewegbares Elektrodenelement (52; 282) umfasst.

12. Chirurgisches System nach einem der voranstehenden Ansprüche, **dadurch gekennzeichnet, dass** mindestens eine der HF-Elektroden (28, 29) in mindestens zwei Elektrodensegmente (30, 31) unterteilt ist und dass die mindestens zwei Elektrodensegmente (30, 31) gegeneinander elektrisch isoliert sind.

13. Chirurgisches System nach einem der voranstehenden Ansprüche, **dadurch gekennzeichnet, dass** das Instrument (12) eine Betätigungseinrichtung (76) umfasst zum Bewegen der Werkzeugelemente (14, 16'; 16"; 16"') relativ zueinander.

14. Chirurgisches System nach einem der voranstehenden Ansprüche, **dadurch gekennzeichnet, dass** mindestens eine der HF-Elektroden (28, 29) in eine Mehrzahl von Elektrodensegmente (30, 31) unterteilt ist.

## Claims

1. Surgical system (10) for connecting tubular parts of body tissue, for end-to-end, side-to-end or side-to-side anastomosis, comprising:
a surgical instrument (12) having
a first ring-shaped or plate-shaped tool element (14) and a second ring-shaped or plate-shaped tool element (16'; 16"; 16"') which are movable relative to each other and each of which comprises a ring-shaped, self-contained high-frequency electrode (28, 29) having a one-part or multi-part configuration, the high-frequency electrode (28, 29), in an approach position of the tool elements (14, 16'; 16"; 16'"), defining a minimum distance (58) from each other, lying opposite each other and facing each other, wherein
the instrument (12) comprises a shaft (24) at the distal end of which the first tool element (14) is arranged or formed,
**characterized in that**
**in that** the second tool element (16'; 16"; 16'") is adapted to be moved from an operating position, in which it is adapted for movement into the approach position, into a removal position and/or vice versa, and
a surface area of a perpendicular projection (232) of the second tool element (16') onto a projection plane (234) extending perpendicularly to the shaft direction (54) in the region of the second tool element (16') being smaller in the removal position than in the operating position.

2. Surgical system in accordance with claim 1, **characterized in that** the second tool element (16'; 16"; 16'") is mounted for movement on a holding member (84'; 84"; 84"').

3. Surgical system in accordance with claim 2, **characterized in that** the second tool element (16'; 16"; 16"') is mounted for pivotal movement about a pivot axis (242; 284; 344) which extends transversely, in particular, perpendicularly, to a holding member longitudinal axis (54) defined by the holding member (84'; 84"; 84'").

4. Surgical system in accordance with claim 2 or 3, **characterized by** a folding mechanism (240; 270; 312) for transferring the second tool element (16'; 16"; 16"') from the operating position into the removal position.

5. Surgical system in accordance with claim 4, **characterized in that** the folding mechanism (240; 270; 312) comprises a force transmitting element (220; 268; 318) for transmitting an actuating force onto the second tool element (16'; 16"; 16'") to transfer the second tool element (16'; 16"; 16'") from the operating position into the removal position and/or vice versa.

6. Surgical system in accordance with claim 5, **characterized in that** the force transmitting element (220; 268; 318) and the holding member (84'; 84"; 84"') are configured for displacement and/or rotation and/or screwing relative to each other.

7. Surgical system in accordance with claim 5 or 6, **characterized by** an actuating mechanism (76) coupled with the folding mechanism (240; 270; 318) and/or the force transmitting element (220; 268; 318) and/or the holding member (84'; 84"; 84'") for actuating the folding mechanism (240; 270; 318) and/or moving the force transmitting element (220; 268; 318) and/or the holding member (84'; 84"; 84'") relative to the shaft.

8. Surgical system in accordance with one of the claims 5 to 7, **characterized in that** the second tool element (16'; 16'") and the force transmitting element (220; 318) are articulatedly coupled to each other by at least one articulation member (194; 314).

9. Surgical system in accordance with one of the preceding claims, **characterized in that** the second tool element (16') is of two-part or multi-part configuration.

10. Surgical system in accordance with one of the preceding claims, **characterized in that** the second tool element (16') comprises at least two tool element parts (180, 182) which are movable relative to each other during the transition from the operating position to the removal position.

11. Surgical system in accordance with one of the preceding claims, **characterized in that** the second tool element (16'; 16"; 16"') comprises an electrode element (52; 282) movable in the shaft direction (54) and movable in the direction towards and away from the first tool element (14).

12. Surgical system in accordance with one of the preceding claims, **characterized in that** at least one of the high-frequency electrodes (28, 29) is divided up into at least two electrode segments (30, 31), and the at least two electrode segments (30, 31) are electrically insulated from each other.

13. Surgical system in accordance with one of the preceding claims, **characterized in that** the instrument (12) comprises an actuating device (76) for moving the tool elements (14, 16'; 16"; 16"') relative to each other.

14. Surgical system in accordance with one of the preceding claims, **characterized in that** at least one of the high-frequency electrode (28, 29) is divided up into a plurality of electrode segments (30, 31).

## Revendications

1. Système chirurgical (10) servant à relier des parties de tissu corporel de forme tubulaire lors d'une anastomose d'extrémité à extrémité, de côté à extrémité ou de côté à côté, comprenant
un instrument chirurgical (12) pourvu d'un premier élément d'outil (14) réalisé de manière à présenter une forme d'anneau ou une forme de plaque et d'un deuxième élément d'outil (16' ; 16" ; 16'") réalisé de manière à présenter une forme d'anneau ou une forme de plaque, lesquels peuvent être déplacés l'un par rapport à l'autre et comprennent respectivement une électrode HF (28, 29) réalisée de manière à présenter une forme annulaire, en une ou plusieurs parties, fermée sur elle-même, qui définissent, dans une position de rapprochement des éléments d'outil (14, 16' ; 16" ; 16'"), une distance minimale (58) l'une par rapport à l'autre, se font face l'une l'autre et pointent en direction l'une de l'autre, dans lequel
l'instrument (12) présente une tige (24), au niveau de l'extrémité distale de laquelle le premier élément d'outil (14) est disposé ou réalisé,
**caractérisé en ce que**
le deuxième élément d'outil (16' ; 16" ; 16'") peut être amené depuis une position de fonctionnement, dans laquelle il peut être amené dans la position de rapprochement, dans une position d'éloignement et/ou inversement, et
dans la position d'éloignement, une zone de surface d'une projection (232) perpendiculaire du deuxième élément d'outil (16') sur un plan de projection (234), qui s'étend de manière perpendiculaire par rapport au sens de tige (54) dans la zone du deuxième élément d'outil (16'), est plus petite que dans la position de fonctionnement.

2. Système chirurgical selon la revendication 1, **caractérisé en ce que** le deuxième élément d'outil (16' ; 16" ; 16'") est monté de manière à pouvoir être déplacé au niveau d'un membre de retenue (84' ; 84" ; 84'").

3. Système chirurgical selon la revendication 2, **caractérisé en ce que** le deuxième élément d'outil (16' ; 16" ; 16'") est monté de manière à pouvoir pivoter autour d'un axe de pivotement (242 ; 284; 344), lequel s'étend de manière transversale, en particulier de manière perpendiculaire, par rapport à un axe longitudinal de membre de retenue (54) défini par le membre de retenue (84' ; 84" ; 84"').

4. Système chirurgical selon la revendication 2 ou 3, **caractérisé par** un mécanisme de pliage (240 ; 270 ; 312) servant à transférer le deuxième élément d'outil (16' ; 16" ; 16'") de la position de fonctionnement dans la position d'éloignement.

5. Système chirurgical selon la revendication 4, **caractérisé en ce que** le mécanisme de pliage (240; 270; 312) comprend un élément de transmission de force (220; 268; 318) servant à transférer une force d'actionnement sur le deuxième élément d'outil (16' ; 16" ; 16'") servant à transférer ce dernier de la position de fonctionnement dans la position d'éloignement, et/ou inversement.

6. Système chirurgical selon la revendication 5, **caractérisé en ce que** l'élément de transmission de force (220 ; 268 ; 318) et le membre de retenue (84' ; 84" ; 84'") sont réalisés de manière à pouvoir être coulissés et/ou tournés et/ou vissés l'un par rapport à l'autre.

7. Système chirurgical selon la revendication 5 ou 6, **caractérisé par** un mécanisme d'actionnement (76) couplé au mécanisme de pliage (240; 270; 318) et/ou à l'élément de transmission de force (220 ; 268 ; 318) et/ou au membre de retenue (84' ; 84" ; 84'"), servant à actionner le mécanisme de pliage (240 ; 270 ; 318) et/ou servant à déplacer l'élément de transmission de force (220 ; 268 ; 318) et/ou le membre de retenue (84' ; 84" ; 84'") par rapport à la tige.

8. Système chirurgical selon l'une quelconque des revendications 5 à 7, **caractérisé en ce que** le deuxième élément d'outil (16' ; 16'") et l'élément de transmission de force (220 ; 318) sont couplés l'un à l'autre de manière articulée par l'intermédiaire d'au moins un membre d'articulation (194 ; 314).

9. Système chirurgical selon l'une quelconque des revendications précédentes, **caractérisé en ce que** le deuxième élément d'outil (16') est réalisé en deux ou en plusieurs parties.

10. Système chirurgical selon l'une quelconque des revendications précédentes, **caractérisé en ce que** le deuxième élément d'outil (16') comprend au moins deux parties d'élément d'outil (180, 182), qui peuvent être déplacées l'une par rapport à l'autre lors du passage de la position de fonctionnement dans la position d'éloignement.

11. Système chirurgical selon l'une quelconque des revendications précédentes, **caractérisé en ce que** le deuxième élément d'outil (16' ; 16" ; 16'") comprend un élément formant électrode (52 ; 282) pouvant être déplacé dans le sens de tige (54) et pouvant être déplacé en direction du premier élément d'outil (14) et de manière à s'en éloigner.

12. Système chirurgical selon l'une quelconque des revendications précédentes, **caractérisé en ce qu'**au moins une des électrodes HF (28, 29) est divisée en au moins deux segments d'électrode (30, 31), et **en ce que** les au moins deux segments d'électrode (30, 31) sont isolés électriquement les uns des autres.

13. Système chirurgical selon l'une quelconque des revendications précédentes, **caractérisé en ce que** l'instrument (12) comprend un dispositif d'actionnement (76) servant à déplacer les éléments d'outil (14, 16' ; 16" ; 16'") les uns par rapport aux autres.

14. Système chirurgical selon l'une quelconque des revendications précédentes, **caractérisé en ce qu'**au moins une des électrodes HF (28, 29) est divisée en une multitude de segments d'électrode (30, 31).
